(19) Europäisches Patentamt
European Patent Office
Office européen des brevets

(11) EP 1 753 725 B1

(12) # FASCICULE DE BREVET EUROPEEN

(45) Date de publication et mention de la délivrance du brevet:
**27.02.2013 Bulletin 2013/09**

(21) Numéro de dépôt: **05772938.6**

(22) Date de dépôt: **24.05.2005**

(51) Int Cl.:
***C07D 217/02*** (2006.01) ***C07D 217/04*** (2006.01)
***C07D 217/06*** (2006.01) ***C07D 401/12*** (2006.01)
***C07D 405/06*** (2006.01) ***C07D 409/06*** (2006.01)
***A61K 31/4725*** (2006.01) ***A61K 31/472*** (2006.01)
***A61K 31/496*** (2006.01) ***A61K 31/5377*** (2006.01)
***A61P 3/04*** (2006.01) ***A61P 3/10*** (2006.01)
***A61P 25/00*** (2006.01)

(86) Numéro de dépôt international:
**PCT/FR2005/001279**

(87) Numéro de publication internationale:
**WO 2005/118547 (15.12.2005 Gazette 2005/50)**

(54) **DERIVES DE TETRAHYDROISOQUINOLILSULFONAMIDES, LEUR PREPARATION ET LEUR UTILISATION EN THERAPEUTIQUE**

TETRAHYDROISOCHINOLINSULFONAMID-DERIVATE, DEREN HERSTELLUNG UND THERAPEUTISCHE VERWENDUNG

TETRAHYDROISOQUINOLINE SULFONAMIDE DERIVATIVES, THE PREPARATION THEREOF, AND THE USE OF THE SAME IN THERAPEUTICS

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **25.05.2004 FR 0405607**

(43) Date de publication de la demande:
**21.02.2007 Bulletin 2007/08**

(73) Titulaire: **SANOFI**
**75008 Paris (FR)**

(72) Inventeurs:
- **DIAZ MARTIN, Juan Antonio**
**E-28016 Madrid (ES)**
- **JIMENEZ BARGUENO, Maria Dolores**
**E-28100 Alcobendas (ES)**

(74) Mandataire: **Kugel, Dominique et al**
**Sanofi-Aventis**
**Département Brevets**
**174 Avenue de France**
**75013 Paris (FR)**

(56) Documents cités:
**WO-A-02/076925** **WO-A-03/055848**
**WO-A-2004/019935**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 753 725 B1

## Description

La présente invention a pour objet des dérivés de sulfonamides, leurs préparations et leurs applications en thérapeutique, notamment dans le traitement des troubles améliorés par modulation du récepteur $H_3$ de l'histamine, tels que l'obésité, le diabète et des maladies du système nerveux central telles que troubles de la vigilance et du sommeil.
La demande de brevet WO 03/055848 décrit des dérivés urées antagonistes de VR1. La demande de brevet WO 2004/019935 décrit des dérivés 3-(sulfonamidoéthyl)-indoles présentant une activité glucocorticoïde.
La demande de brevet WO 02/076925 décrit des dérivés isoquinolyle éthers, antagonistes du récepteur H3 de l'histamine.
En conséquence la présente invention a pour premier objet les composés répondant à la formule I

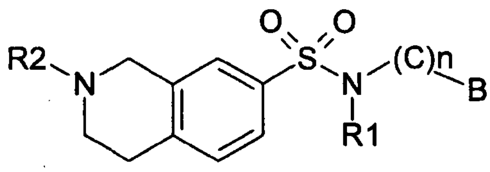

I

dans laquelle:

**n** peut prendre une valeur de 1 à 6 ;

**-(C)n-** représente un groupe -$C_{1-6}$ alkylidène, éventuellement substitué par 1 à 4 substituants choisis parmi un atome d'halogène, un groupe hydroxy, nitro, cyano, amino, $C_{1-3}$ monoalkylamino, $C_{2-6}$ dialkylamino ou $C_{1-3}$ alcoxy ;

**R1** représente

- Un atome d'hydrogène
- Un groupe $C_{1-6}$ alkyle.

**R2** représente

- Un atome d'hydrogène,
- a Un groupe $C_{1-6}$ alkyle ou $C_{3-6}$ cycloalkyle éventuellement substitués par 1 à 4 substituants choisis parmi un atome d'halogène, un groupe hydroxy, nitro, cyano, amino, $C_{1-3}$ monoalkylamino, $C_{2-6}$ dialkylamino, $C_{1-2}$ perhalogénoalkyle, $C_{1-3}$ halogénoalkyle, $C_{1-3}$ alcoxy, $C_{3-6}$ cycloalkyle, un hétéroaryle monocyclique tel qu'un thiényle, furyle ou pyrrolyle ou un aryle, tel qu'un phényle ou un naphtyle; l'aryle étant éventuellement substitué par 1 à 4 substituants choisis parmi un atome d'halogène, un groupe hydroxy, nitro, cyano, amino, $C_{1-3}$ monoalkylamino, $C_{2-6}$ dialkylamino, $C_{1-3}$ alkyle, $C_{1-2}$ perhalogénoalkyle, $C_{1-3}$ halogénoalkyle, $C_{1-3}$ alcoxy ou un groupe $C_{1-3}$ alkylidènedioxy ;

**B** représente

- NR3R4,
  - R3 et R4 représentant, indépendamment l'un de l'autre, un groupe $C_{1-6}$ alkyle, un atome d'hydrogène; ou
  - R3 et R4 représentent ensemble un groupe $C_{1-6}$ alkylidène, un groupe $C_{2-8}$ alkenylidène, un groupe $C_{1-3}$ alkylidène-O-$C_{1-3}$ alkylidène, ou un groupe $C_{1-3}$ alkylidène-N(R5)-$C_{1-3}$ alkylidène où R5 représente un atome d'hydrogène, un groupe $C_{1-3}$ alkyle ou $C_{1-6}$ alkylcarbonyle, ces groupes $C_{1-3}$ alkyle et $C_{1-6}$ alkylcarbonyle pouvant être substitués par un atome d'halogène, un groupe hydroxy, $C_{1-3}$ alcoxy, nitro, cyano ou amino; ou
- un aminocycle, lié par un carbone au groupe -NR1-(C)n-, choisi parmi aziridine, azétidine, pyrrolidine, pipéridine et morpholine ;

les groupes R3, R4, ainsi que l'aminocycle étant éventuellement substitués par 1 à 4 substituants choisis parmi un phényle, un benzyle, un atome d'halogène, un groupe hydroxy, nitro, cyano, amino, $C_{1-3}$ monoalkylamino, $C_{2-6}$ dialkylamino, $C_{1-3}$ alkyle, ou $C_{1-3}$ alcoxy ; et l'atome d'azote éventuellement substitué par un $C_{1-3}$ alkyle, à l'exclusion du

composé dans lequel $R_1$ et $R_2$ représentent des atomes d'hydrogène, B représente un groupe diméthylamino et -(C)n- représente un groupe éthylidène.

Dans le cadre de la présente invention, on entend par :

- $C_{x-z}$, une chaîne carbonée pouvant avoir de x à z atomes de carbone, par exemple $C_{1-3}$ indique une chaîne carbonée pouvant avoir de 1 à 3 atomes de carbone;
- alkyle, un groupe aliphatique saturé linéaire ou ramifié; par exemple, un groupe $C_{1-4}$ alkyle représente une chaîne carbonée de 1 à 6 atomes de carbone saturée, linéaire ou ramifiée, plus particulièrement un radical méthyle, éthyle, propyle, isopropyle, butyle, isobutyle, tertbutyle, etc. ; le terme $C_{x-y}$ alkylidène désignant un groupe $C_{x-y}$ alkyle, linéaire ou ramifié, divalent; le terme $C_{2-8}$ alkenylidène désignant un groupe $C_{x-y}$ alkyle, insaturé, linéaire ou ramifié, divalent ;
- $C_{x-y}$ alcoxy, un groupe alkyloxy à chaîne aliphatique saturée, linéaire ou ramifiée, comportant x à y atomes de carbone;
- atome d'halogène, un fluor, un chlore, un brome ou un iode ;
- $C_{1-3}$ monoalkylamino, un amino mono substitué par un groupe $C_{1-3}$ alkyle ;
- $C_{2-6}$ dialkylamino, un amino di-substitué par deux groupes $C_{1-3}$ alkyle ;
- $C_{1-2}$ perhalogénoalkyle, un groupe $C_{1-2}$ alkyle dans lequel tous les atomes d'hydrogène sont substitués par des atomes d'halogène ;
- $C_{1-3}$ halogénoalkyle, un groupe $C_{1-3}$ alkyle dans lequel au moins un atome d'hydrogène sont substitués par un atome d'halogène.

Les composés de formule I peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule générale I peuvent se présenter sous forme de bases libres ou de sels d'addition à des acides, qui font également partie de l'invention. Ces sels, selon la présente invention, comprennent ceux avec des acides pharmaceutiquement acceptables mais aussi ceux avec des acides minéraux ou organiques qui permettent une séparation ou une cristallisation convenable des composés de formule I. Ces sels peuvent être préparés, selon des méthodes connues de l'homme du métier, par exemple, par réaction du composé de formule I sous forme de base avec l'acide dans un solvant approprié, tel qu'une solution alcoolique ou un solvant organique, puis séparation du milieu qui le contient par évaporation du solvant ou par filtration.

Les composés de formule I peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

D'autre part, dans le cadre de la présente invention, on entend par groupe protecteur Pg, un groupe qui permet d'une part de protéger une fonction réactive telle qu'un hydroxy, ou une amine pendant une synthèse et d'autre part de régénérer la fonction réactive intacte en fin de synthèse. Des exemples de groupes protecteurs ainsi que les méthodes de protection et déprotection sont données dans «Protective groups in Organic Synthesis 3th Ed.», Greene et Wuts (John Wiley & Sons, Inc., New York, 1999).

La présente invention a également pour objet les composés choisis parmi les sous-groupes suivants, dans lesquels:

- **n** est égal à 2, 3 ou 4 ; et/ou
- **R1** représente un atome d'hydrogène ou un groupe $C_{1-2}$ alkyle; et/ou
- **R2** représente un atome d'hydrogène, un groupe $C_{1-4}$ alkyle ou $C_{5-6}$ cycloalkyle éventuellement substitués par 1 à 4 substituants choisis parmi un phényle, un groupe $C_{3-6}$ cycloalkyle, $C_{1-2}$ perhalogénoalkyle, $C_{1-3}$ halogénoalkyle ou $C_{1-3}$ alcoxy ; le phényle étant éventuellement substitués par 1 à 4 substituants choisis parmi un atome d'halogène, un groupe hydroxy, nitro, cyano, amino, $C_{1-3}$ monoalkylamino, $C_{2-6}$ dialkylamino, $C_{1-3}$ alkyle, $C_{1-2}$ perhalogénoalkyle, $C_{1-3}$ halogénoalkyle, $C_{1-3}$ alcoxy ou un groupe $C_{1-3}$ alkylidènedioxy ; et/ou
- **B** représente

  - NR3R4,

    - R3 et R4 représentant, indépendamment l'un de l'autre, un groupe $C_{1-4}$ alkyle; ou
    - lorsque R3 et R4 représentent ensemble un groupe $C_{1-6}$ alkylidène, un groupe $C_{2-8}$ alkenylidène, $C_{1-3}$ alkylidène-O-$C_{1-3}$ alkylidène, ou $C_{1-3}$ alkylidène-N(R5)-$C_{1-3}$ alkylidène , B représente un groupe :

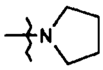 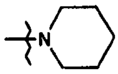 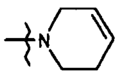 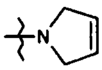 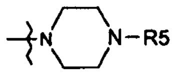 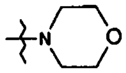 ;

ou

- un aminocycle lié par un carbone au groupe -NR1-(C)n, choisi parmi aziridine, azetidine, pyrrolidine, pipéridine et morpholine;

les groupes R3, R4 et R5 ainsi que l'aminocycle étant éventuellement substitués ; et plus spécifiquement le sous-groupe où à la fois n, R1, R2 et B sont tels que définis ci-dessus.

Plus particulièrement, lorsque B représente NR3R4 et R3 et R4 forment ensemble un groupe $C_{1-6}$ alkylidène, un groupe $C_{2-8}$ alkenylidène, $C_{1-3}$ alkylidène-O-$C_{1-3}$ alkylidène ou $C_{1-3}$ alkylidène-N(R5)-$C_{1-3}$ alkylidène ou lorsque **B** représente un aminocycle alors, B est choisi parmi les groupes suivants:

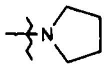 ou 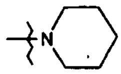 ou 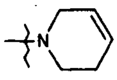 ou 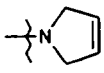 ou 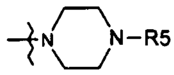 ou 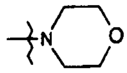

ou

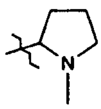 ou 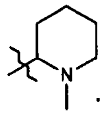 .

Un autre objet de la présente invention, concernent les composés suivants:

1. N-[3-(diéthylamino)propyl]-1,2,3,4-tétrahydroisoquinoline-7-sulfonamide;
2. (+/-)-N-[2-(1-méthylpyrrolidin-2-yl)éthyl]-1,2,3,4-tétrahydroisoquinoline-7-sulfonamide;
3. N-[3-(diéthylamino)propyl]-2-méthyl-1,2,3,4-tétrahydroisoquinoline-7-sulfonamide;
4. N-[3-(diéthylamino)propyl]-N-méthyl-1,2,3,4-tétrahydroisoquinoline-7-sulfonamide;
5. 2-benzyl-N-[3-(diéthylamino)propyl]-1,2,3,4-tétrahydroisoquinoline-7-sulfonamide;
6. 2-(cyclopropylméthyl)-N-[3-(diéthylamino)propyl]-1,2,3,4-tétrahydroisoquinoline-7-sulfonamide;
7. 2-(cyclohéxylmethyl)-N-[3-(diéthylamino)propyl]-1,2,3,4-tétrahydroisoquinoline-7-sulfonamide;
8. (+/-)-N-[3-(2-méthylpiperidin-1-yl)propyl]-1,2,3,4-tétrahydroisoquinoline-7-sulfonamide;
9. N-[3-(3,6-dihydropyridin-1(2*H*)-yl)propyl]-1,2,3,4-tétrahydroisoquinoline-7-sulfonamide;
10. N-[3-(diéthylamino)propyl]-2-isopropyl-1,2,3,4-tétrahydroisoquinoline-7-sulfonamide;
11. N-[3-(diéthylamino)propyl]-2-(2-thiénylméthyl)-1,2,3,4-tetrahydroisoquinoline-7-sulfonamide;
12. N-[3-(diéthylamino)propyl]-2-(3-thiénylméthyl)-1,2,3,4-tetrahydroisoquinoline-7-sulfonamide;
13. N-[3-(2,5-dihydro-1*H*-pyrrol-1-yl)propyl]-1,2,3,4-tétrahydroisoquinoline-7-sulfonamide;
14. 2-cyclohéxyl-N-[3-(diéthylamino)propyl]-1,2,3,4-tétrahydroisoquinoline-7-sulfonamide;
15. (+/-)-2-(cyclohéxylmethyl)-N-[2-(1-méthylpyrrolidin-2-yl)éthyl]-1,2,3,4-tétrahydroisoquinoline-7-sulfonamide;
16. N-[3-(2,5-dihydro-1*H*-pyrrol-1-yl)propyl]-2-méthyl-1,2,3,4-tétrahydroisoquinoline-7-sulfonamide;
17. N-[3-(4-benzylpiperazin-1-yl)propyl]-1,2,3,4-tétrahydroisoquinoline-7-sulfonamide;
18. N-(3-pyrrolidin-1-ylpropyl)-1,2,3,4-tétrahydroisoquinoline-7-sulfonamide;
19. N-(3-morpholin-4-ylpropyl)-1,2,3,4-tétrahydroisoquinoline-7-sulfonamide;
20. N-[3-(diméthylamino)propyl]-1,2,3,4-tétrahydroisoquinoline-7-sulfonamide;
21. 2-(cyclohéxylmethyl)-N-(3-pyrrolidin-1-ylpropyl)-1,2,3,4-tétrahydroisoquinoline-7-sulfonamide;
22. (+/-)-2-(cyclopropylméthyl)-N-[2-(1-méthylpyrrolidin-2-yl)éthyl]-1,2,3,4-tétrahydroisoquinoline-7-sulfonamide;
23. (+/-)-2-benzyl-N-[2-(1-méthylpyrrolidin-2-yl)éthyl]-1,2,3,4-tétrahydroisoquinoline-7-sulfonamide;
24. (+/-)-2-(4-isopropylbenzyl)-N-[2-(1-méthylpyrrolidin-2-yl)éthyl]-1,2,3,4-tétrahydroisoquinoline-7-sulfonamide;
25. (+/-)-2-(1,3-benzodioxol-5-ylméthyl)-N-[2-(1-méthylpyrrolidin-2-yl)éthyl]-1,2,3,4-tétrahydroisoquinoline-7-sulfonamide;
26. (+)-N-[2-(1-méthylpyrrolidin-2-yl)éthyl]-1,2,3,4-tétrahydroisoquinoline-7-sulfonamide;
27. (-)-N-[2-(1-méthylpyrrolidin-2-yl)éthyl]-1,2,3,4-tétrahydroisoquinoline-7-sulfonamide;
28. (+)-2-(cyclohéxylmethyl)-N-[2-(1-méthylpyrrolidin-2-yl)éthyl]-1,2,3,4-tétrahydroisoquinoline-7-sulfonamide;
29. (-)-2-(cyclohéxylmethyl)-N-[2-(1-méthylpyrrolidin-2-yl)éthyl]-1,2,3,4-tétrahydroisoquinoline-7-sulfonamide;

30. (+/-)-2-(4-bromo-benzyl)-)-*N*-[2-(1-méthylpyrrolidin-2-yl)éthyl]-1,2,3,4-tétrahydroisoquinoline-7-sulfonamide
31. (+/-)-2-(2,5-dimétoxy-benzyl)-)-*N*-[2-(1-méthylpyrrolidin-2-yl)éthyl]-1,2,3,4-tétrahydroisoquinoline-7-sulfonamide
32. (+/-)-2-(2-méthyl-butyl)-)-*N*-[2-(1-méthylpyrrolidin-2-yl)éthyl]-1,2,3,4-tétrahydroisoquinoline-7-sulfonamide
33. (+/-)-2-(3-méthoxy-benzyl)-)-*N*-[2-(1-méthylpyrrolidin-2-yl)éthyl]-1,2,3,4-tétrahydroisoquinoline-7-sulfonamide et
34. (+/-)-2-(3,5-diméthyl-benzyl)-)-*N*-[2-(1-méthylpyrrolidin-2-yl)éthyl]-1,2,3,4-tétrahydroisoquinoline-7-sulfonamide

La présente invention a pour second objet des procédés de préparation des composés de formule I selon l'invention.

Ainsi, les composés de formule I peuvent être préparés selon le procédé représenté dans le schéma 1.

Schéma 1

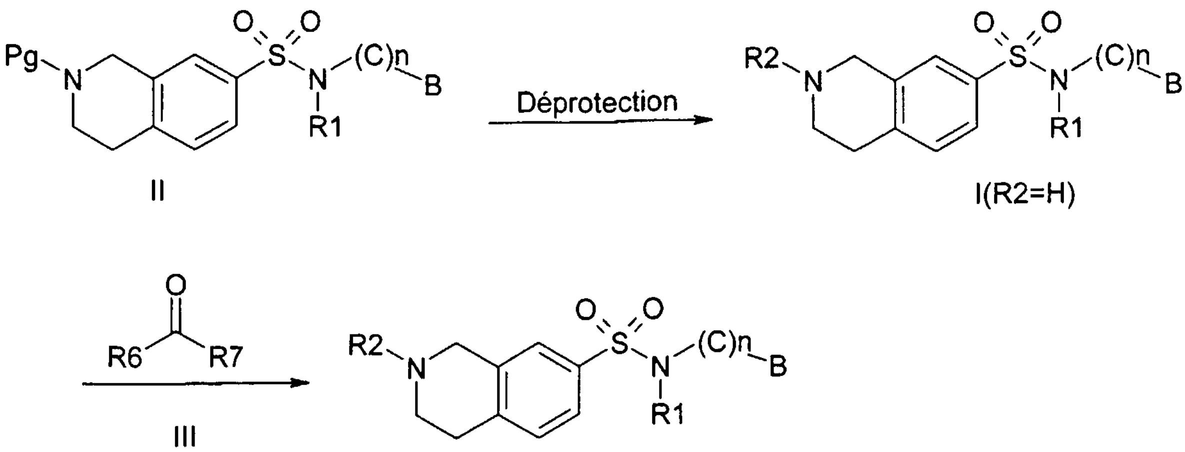


II

I(R2=H)

III

I (R2 différent de H)

Selon le procédé du schéma 1, les composés de formule I, dans laquelle R2 est différent d'un atome d'hydrogène, sont préparés par amino-réduction, en faisant réagir une amine secondaire de formule I, dans laquelle R2 représente H, avec un aldéhyde ou une cétone de formule III, où R6 et R7, après réaction forment ensemble R2 tel que défini dans la formule I. Les composés de formule I, où R2 représente un atome d'hydrogène, peuvent être obtenus par déprotection des composés de formule II, selon des méthodes classiques connues de l'homme du métier. Par exemple, les composés de formule II, lorsque Pg est un groupe trifluoroacétyle, peuvent être déprotégés en présence d'une base telle que, par exemple, la carbonate de sodium ou de potassium, l'ammoniac ou l'hydroxyde de baryum dans un solvant protique, tel que l'eau, le méthanol ou un mélange de ces solvants, à une température comprise entre 0 et 100°C. Alternativement, la déprotection des composés de formule II, lorsque Pg est un groupe trifluoroacétyle, peut être réalisée en présence d'un acide tel que, par exemple, l'acide chlorhydrique dans un solvant protique ou aprotique, tel que l'eau, le méthanol, l'éthanol, l'acétate d'éthyle ou un mélange de ces solvants, à une température comprise entre 0 et 100°C. Des illustrations du procédé sont données dans les exemples

Les composés de départ de formule II peuvent être préparés selon le schéma 2 ou peuvent être synthétisés par des méthodes classiques connues de l'homme du métier.

Schéma 2

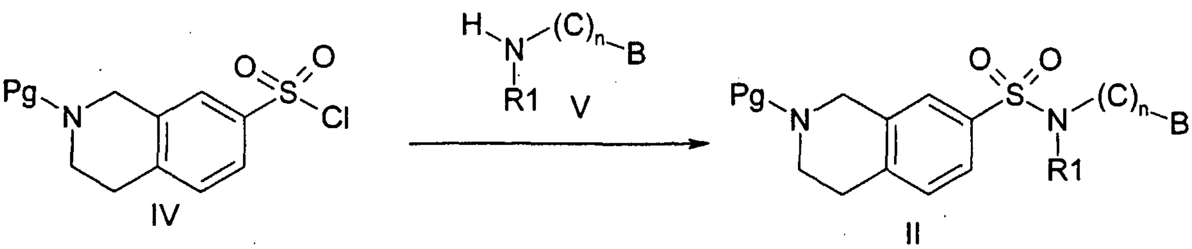

IV

V

II

Selon ce schéma, les composés de formule II, dans laquelle n, R1 et B sont tels que défini dans la formule I, peuvent être préparés par réaction d'un amine de formule V, dans laquelle R1 et B sont tels que défini dans la formule I, avec un chlorure de sulfonyle de formule IV, dans laquelle Pg représente un groupe protecteur approprié tel que par exemple, une trifluoroacetamide, pour former un dérivé de type sulfonamide de formule II selon des méthodes classiques connues de l'homme du métier, par exemple, la réaction peut être réalisée dans un solvant protique ou aprotique, tel que le tétrahydrofurane, le.dichlorométhane, l'acétate d'éthyle, la N,N-diméthylformamide, l'acétonitrile ou un mélange de ces solvants, à une température comprise entre 0 et 100°C, en présence d'une base telle que par exemple la carbonate de potassium, de sodium, la diisopropyléthylamine ou la triéthylamine. Alternativement, les composés de formule II peuvent être préparés par une réaction de type Mitsunobu, selon le schéma 3.

## Schéma 3

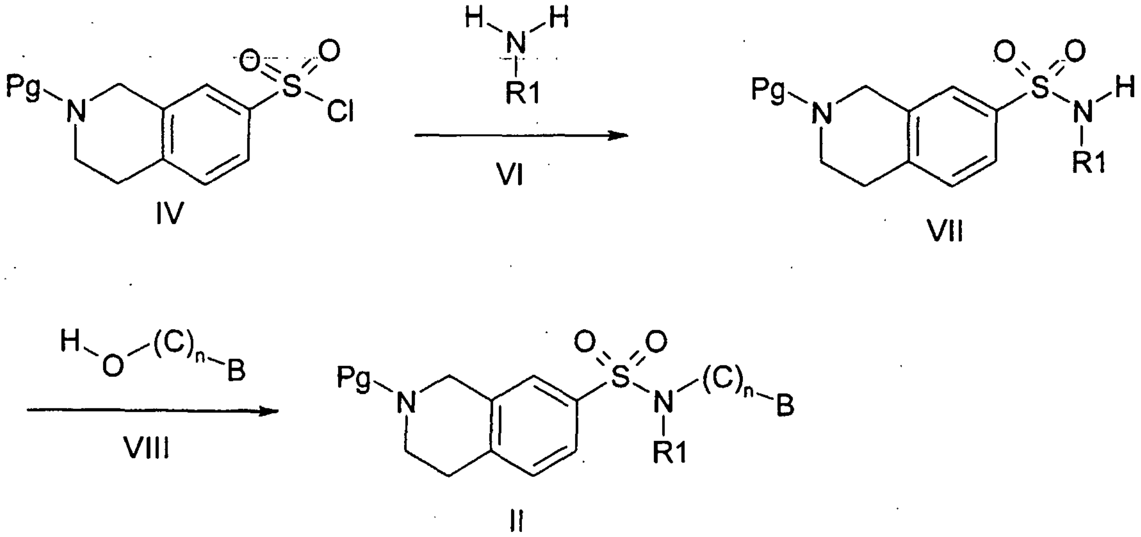

IV VI VII VIII II

Selon cette alternative, on fait réagir une sulfonamide de formule VII, dans laquelle R1 est tel que défini dans la formule I, avec un amino-alcool de formule VIII, dans laquelle n et B sont définis comme précédemment. La réaction peut être réalisée de façon classique en présence de réactifs de Mitsunobu tel qu'un azodérivé, par exemple, le diéthylazodicarboxylate, le diisopropylazodicarboxylate, le diterbutylazodicarboxylate, la 1,1'-(azodicarbonyl)dipiperidine ou la *N,N,N',N'*-tétraméthylazodicarboxamide et une phosphine, par exemple, la triphénylphosphine ou la tributylphosphine. La réaction peut être réalisée dans un solvant aprotique, tel que le tétrahydrofurane, ou le dioxane ou un mélange de ces solvants, à une température comprise entre 0 et 100°C, pour donner le composé de formule II. La sulfonamide de formule VII dans laquelle R1 est tel que défini dans la formule I, peut être préparée par réaction d'un amine de formule VI, dans laquelle R1 est tel que défini dans la formule I avec un chlorure de sulfonyle de formule IV, dans laquelle Pg représente un groupe protecteur approprié tel que par exemple, une trifluoroacetamide, selon des méthodes classiques connues de l'homme du métier, par exemple, la réaction peut être réalisée dans un solvant protique ou aprotique, tel que le tétrahydrofurane, le dichlorométhane, l'acétate d'éthyle, la N,N-diméthylformamide, l'acétonitrile ou un mélange de ces solvants, à une température comprise entre 0 et 100°C, en présence d'une base telle que, par exemple la carbonate de potassium, de sodium, la diisopropyléthylamine ou la triéthylamine. Les composés de départ IV et les amines de formule V et VIII sont directement disponibles dans le commerce, peuvent être synthétisés par des méthodes classiques connues de l'homme du métier ou sont connus dans la littérature.

Par exemple, les diamines de formule V, dans laquelle n est égal à 3, peuvent être préparés selon le schéma 4.

## Schéma 4

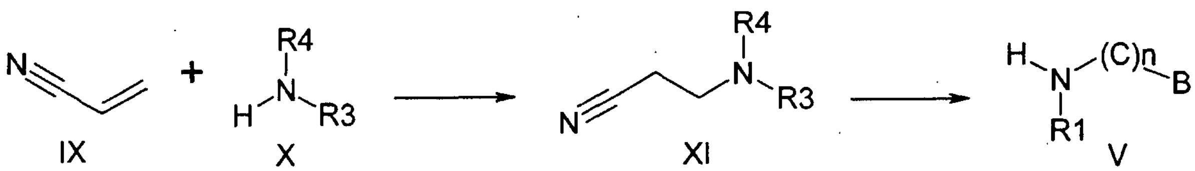

IX X XI V

Selon ce procédé les composés de formule V, dans laquelle n est égal à 3, R1 représente un atome d'hydrogène et B représente un groupe amine, peuvent être préparés par réaction d'adition d'une amine de formule X, dans laquelle R3 et R4 sont définis comme précédemment, avec l'acrylonitrile de formule IX, pour former un dérivé de type aminonitrile de formule XI, selon des méthodes classiques connues de l'homme du métier, suivi par une réduction du nitrile. La réduction peut être réalisée selon des méthodes connues de l'homme du métier, par exemple en présence d'hydrure de diisobutylaluminium à une température comprise entre -70°C et 40°C dans un solvant aprotique tel que le dichlorométhane, le toluène ou un mélange de ces solvants; la réduction peut être réalisée aussi en présence d'un réducteur tel que l'hydrogène en présence d'un catalyseur tel que le platine, le palladium ou le Nickel-Raney, dans un solvant tel que le méthanol ou l'acétate d'éthyle pour donner le composé de formule V, dans laquelle n est égal à 3, R1 représente un atome d'hydrogène et B représente un groupe amine. Les composés de formule V, dans laquelle R1 représente un groupe $C_{1-6}$ alkyle, peuvent être préparés par alkylation du composé de formule V précédemment obtenu selon des méthodes classiques connues de l'homme du métier.

La présente invention a également comme objet les composés de formule II, lorsque Pg représente un groupe protecteur ou un atome d'hydrogene et $-(C)_n$-- et R' sont tels que définis ci-dessus, en tant qu'intermédiaires pour la préparation du composé de formule I. Exclus sont des composés de formule II dans lesquels R' représente un atome d'hydrogène, B représente un groupe diméthylamino et $-(C)_N$- représente un groupe éthylidène.

Les exemples suivants illustrent les procédés et techniques approprié pour la préparation de cette invention, sans toutefois limiter l'étendue de la revendication.

***Exemple 1- Chlorhydrate de N-[2-(1-méthylpyrrolidin-2-yl)éthyl]-1,2,3,4-tétrahydroisoquinoline-7-sulfonamide***

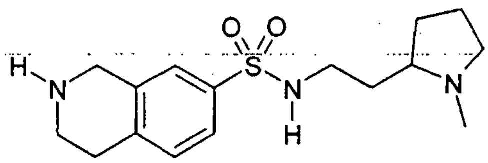

**1.1- Chlorhydrate de (+/-)-N-[2-(1-méthylpyrrolidin-2-yl)éthyl]-2-(2,2,2-trifluoroacétyl)-1,2,3,4-tétrahydroisoquinoline-7-sulfonamide**

Une solution de 5,00 g (0,015 mole) de chlorure de 2-(2,2,2-trifluoroacétyl)-1,2,3,4-tétrahydro-isoquinoline-7-sulphonyle et 2,00 g (0,015 mole) de (+/-)-2-(1-méthyl-pyrrolidin-2-yl)-éthylamine, dans 50 ml de dichlorométhane est agitée pendant la nuit à température ambiante. La solution est concentrée à sec. Le solide formé est purifié par chromatographie sur colonne de gel de silice, avec un mélange de dichlorométhane/méthanol (97:3) employé comme éluant pour donner 4,30 g du produit désiré sous forme d'un solide blanc
Rdt: 62%
PF= amorphe

**1.2- Chlorhydrate de (+/)-N-[2-(1-méthylpyrrolidin-2-yl)éthyl]-1,2,3,4-tétrahydroisoquinoline-7-sulfonamide**

Une solution de 4,30 g (0,0094 mole) du chlorhydrate de 2-(2,2,2-trifluoroacétyl)-1,2,3,4-tétrahydroisoquinoline-7-sulfonamido-[2-(1-méthyl-pyrrolidin-2-yl)-éthyle] dans 50 ml de méthanol saturé de chlorure d'hydrogène est chauffée durant douze heures à 60°C. Le mélange est refroidi et le solide qui s'est formé, est filtré, lavé avec du méthanol et séché. On obtient 2,00 g du produit désiré comme un solide blanc.
Rdt: (65%)
PF= 209-212°C

**$^1$H-NMR (DMSO-$d_6$) $\delta$(ppm):** 7,9 (1 H, t), 7.7 (2H, d), 7,5 (1 H, d), 4,3 (2H, s), 3,6 (5H, m), 3,1 (3H, m) , 2,9 (2H, m), 2,7 (3H, m), 2,1 (1 H, m), 1,9 (3H, m), 1,6 (2H, m), 2,7 (3H, s); 2,9 (2H, t).

**1.3- chlorhydrate de (+) ou (-)-N-[2-(1-méthylpyrrolidin-2-yl)éthyl]-1,2,3,4-tétrahydroisoquinoline-7-sulfonamide**

Le composé obtenu précédemment au 1.2 est séparé par chromatographie préparative sur phase chirale, pour donner ses énantiomères. En effet, la séparation de 15,00 g du chlorhydrate du (+/-)-N-[2-(1-méthylpyrrolidin-2-yl)éthyl]-1,2,3,4-tétrahydroisoquinoline-7-sulfonamide est réalisée avec un système HPLC préparatif PROCHROM LC50, avec une phase stationnaire CHIRALPCK AD et phase mobile formée par isohexane/éthanol/méthanol (80%:10%:10%)+0,2%

de diéthylamine, pour donner 5,39 g du produit dextrogyre, sous forme de poudre blanche, avec une pureté énantiomèrique sur phase chirale de 99,67% et 4,89 g du produit lévogyre, sous forme de poudre blanche, avec une pureté énantiomèrique sur phase chirale de 99,48%. Les deux produits sont transformés en leur correspondant chlorhydrate par traitement avec de l'isopropanol saturé en chlorure d'hydrogène

Enantiomère dextrogyre: P.F:= 114-117°C ; $[\alpha]_D^{20}$ = +16 (c=0,5, méthanol)

Enantiomère lévogyre: P.F= 115-117°C ; $[\alpha]_D^{20}$ =-16 (c=0,5, méthanol)

***Exemple 2- Oxalate (2:1) de (+/-)-2-(cyclohéxylmethyl)-N-[2-(1-méthylpyrrolidin-2-yl)éthyl]-1,2,3,4-tétrahydroisoquinoline-7-sulfonamide]***

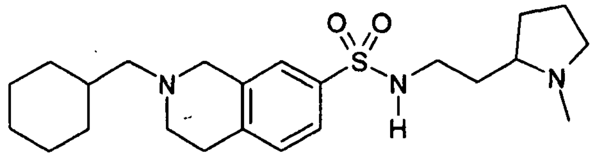

A une solution de 2,02 g (0,0063 mole) de (+/-)-1,2,3,4-tétrahydroisoquinoline-7-sulfonamido-[2-(1-méthylpyrrolidin-2-yl)-éthyle] et 0,70 g (0,0063 mole) de cyclohexanecarboxaldéhyde dans 100 ml de méthanol, on ajoute 0,32 g (0,0003 mole) de palladium sur charbon à 10%. La solution est hydrogénée pendant 24 heures dans un hydrogénateur de Paar à une pression de 3.1 bars. Le catalyseur est éliminé par filtration et la solution filtrée est évaporée à sec. L'huile brute obtenue (2,90 g) est purifiée par chromatographie sur colonne de gel de silice, avec un mélange de dichlorométhane/méthanol (95:5) employé comme éluant. Le produit désiré (1,62 g; 62%) est obtenu sous forme d'une huile.
L'huile précédente est dissoute dans 20 ml d'éthanol, puis 0,77 g (0,0086 mole) d'acide oxalique, dissous dans 15 ml d'éthanol, est ajouté. Le précipité est filtré et lavé avec de l'éthanol froid. On obtient 2,01 g du produit désiré comme un solide blanc.
Rdt: 86%
PF= 142-147°C

**$^1$H-NMR (DMSO-$d_6$) δ(ppm):** 7,5 (2H, m), 7,29 (1 H, m), 3,84 (2H, s), 3,35 (2H, m), 3,1 (1H, m), 2,9 (1 H, m), 2,8 (2H, s), 2,7 (1 H, m), 2,6 (3H, s), 2,5 (2H,m), 2, 1-1,4 (13H, m), 1,15 (4H, m), 0,8 (2H, m).

***Exemple 3- Oxalate (1,5:1) de (+)-2-(cyclohéxylmethyl)-N-[2-(1-méthylpyrrolidin-2-yl)éthyl]-1,2,3,4-tétrahydroisoquinoline-7-sulfonamide***

Selon un procédé similaire à l'exemple 2, avec 1,00 g (0,0031 mole) de (+)-N-[2-(1-méthylpyrrolidin-2-yl)éthyl]-1,2,3,4-tétrahydroisoquinoline-7-sulfonamide et 0,35 g (0,0031 mole) de cyclohexanecarboxaldéhyde dans 50 ml de méthanol comme produit de départ, on obtient 0,46 g de base, qui est transformé dans le correspondant sesquioxalate hydrate comme un solide blanc.
Rdt: 20% PF= 134-140°C

$[\alpha]_D^{20}$ +12 (c=0,5, méthanol)

***Exemple 4- Oxalate (2:1) de (-)-2-(cyclohéxylmethyl)-N-[2-(1-méthylpyrrolidin-2-yl)éthyl]-1,2,3,4-tétrahydroisoquinoline-7-sulfonamide***

Selon un procédé similaire à l'exemple 2, avec 1,00 g (0,0031 mole) de (-)-N-[2-(1-méthylpyrrolidin-2-yl)éthyl]-1,2,3,4-tétrahydroisoquinoline-7-sulfonamide et 0,35 g (0,0031 mole) de cyclohexanecarboxaldéhyde dans 50 ml de méthanol comme produit de départ, on obtient 0,90 g de base, qui est transformé dans le correspondant dioxalate hydrate comme un solide blanc.
Rdt: 27%

PF= 133-138°C $[\alpha]_D^{20}$ = -8 (c=0,5, méthanol)

*Exemple 5- Oxalate (2:1) de 2-benzyl-N-[3-(diéthylamino)propyl]-1,2,3,4-tétrahydroisoquinoline-7-sulfonamide*

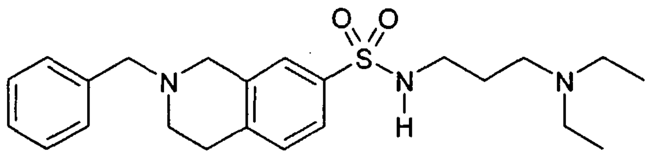

**5.1- Chlorhydrate de N-[2-(3-diéthylamino-propyle)-2-(2,2,2-trifluoroacétyl)-1,2,3,4-tétrahydroisoquinoline-7-sulfonamide**

Une solution de 1,00 g (0.0031 mole) de chlorure de 2-(2,2,2-trifluoroacétyl)-1,2,3,4-tétrahydroisoquinoline-7-sulphonyle et 0,61 g (0,0047 mole) de N,N-diéthyl- N-aminopropyl amine dans 25 ml de dichlorométhane est agitée toute la nuit à température ambiante. La solution est concentrée à sec et l'huile formé est purifié par chromatographie sur colonne de gel de silice, avec un mélange de dichlorométhane/méthanol (97:3), employé comme éluant. On obtient 1,27 g.
Rdt: 97 %
PF= huile

**5.2- Chlorhydrate de N-[3-(diéthylamino)propyl]-1,2,3,4-tétrahydroisoquinoline-7-sulfonamide**

Une solution de 5.39 g (0.013 mole) du chlorhydrate du N-[2-(3-diéthylamino-propyle)-2-(2,2,2-trifluoroacétyl)-1,2,3,4-tétrahydroisoquinoline-7-sulfonamide, dissous dans 60 ml de méthanol saturé en chlorure d'hydrogène, est chauffé durant douze heures à 60°C. Le mélange est refroidi et le solide qui s'est formé, est filtré, lavé avec du méthanol et séché. Le résidu dissout dans une solution aqueuse d'hydroxyde de sodium. La phase aqueuse est extraite plusieurs fois avec de l'éther éthylique. Les phases organiques sont réunies et séchées sur sulfate de magnésium anhydre. L'huile obtenue (2,80 g) est utilisée sans purification additionnelle.
Rdt: 67%
PF= huile

**5.3- Oxalate (2:1) de 2-benzyl-N-[3-(diéthylamino)propyl]-1,2,3,4-tétrahydroisoquinoline-7-sulfonamide**

A une suspension de 0,45 g (0,0014 mole) de N-[3-(diéthylamino)propyl]-1,2,3,4-tétrahydroisoquinoline-7-sulfonamide et 0,149 g (0,0014 mole) de benzaldéhyde dans 12 ml de tétrahydrofurane, sont ajoutés 1,5 ml de l'acide acétique. La suspension est agité durant 1 heure à température ambiante et puis sont ajoutés 0,14 g (0,0021 mole) de cyanoborohydrure de sodium. On agite le mélange pendant la nuit. On concentre à sec et le résidu est traité avec de l'eau et lavé avec de l'éther éthylique. La phase aqueuse est basifiée à pH= 10 et est extraite plusieurs fois avec de l'éther éthylique. Les phases organiques sont réunies et séchées sur de sulfate de magnésium anhydre. L'huile obtenue est purifiée par chromatographie sur colonne de gel de silice, avec un mélange de dichlorométhane/méthanol (95:5) employé comme éluant. Le produit désiré (0,13 g; 22%) est obtenu sous forme d'une huile.

L'huile précédente est dissoute dans 5 ml d'éthanol, puis 0,06 g (0,0007 mole) d'acide oxalique, dissous dans 5 ml d'éthanol, sont ajoutés. Le précipité est filtré et lavé avec de l'éthanol froid. On obtient 0,12 g du produit désiré comme un solide blanc.
Rdt: 63%
PF= 91-103°C

**$^1$H-NMR (DMSO-$d_6$) $\delta$(ppm):** 7.5-7.1 (8H, m), 3.52 (2H, d), 2.9-2.5 (14H, m), 1.5 (2H, m), 0.9 (6H, t)

*Exemple 6- Chlorhydrate de N-[3-(diéthylamino)propyl]-N-méthyl-1,2,3,4-tétrahydroisoquinoline-7-sulfonamide*

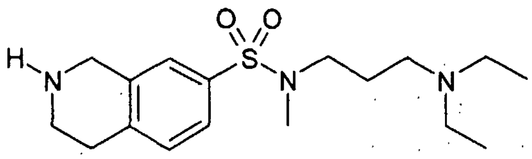

**6.1- N-[2-(3-Diéthylamino-propyle)-N-méthyl-2-(2,2,2-trifluoroacétyl)-1,2,3,4-tétrahydroisoquinoline-7-sulfonamide**

A une solution de 0,91 g (0,0021 mole) de N-[2-(3-diéthylamino-propyle)-2-(2,2,2-trifluoroacétyl)-1,2,3,4-tétrahydroisoquinoline-7-sulfonamide (obtenue selon la méthode décrite à l'étape 5.1- de l'exemple 5) dans 17 ml de diméthylformamide, refroidie à 0°C, est ajouté 0,09 g (0,0022 mole) d'une dispersion d'hydrure de sodium (60%) dans une huile minérale. Le mélange est agité pendant une heure et on ajoute 0,60 g (0,0042 mole) d'iodure de méthyle. Le mélange est agité durant la nuit à température ambiante, la solution est concentrée à sec. Le résidu est traité avec de l'eau. La phase aqueuse est extraite plusieurs fois avec de l'acétate d'éthyle. Les phases organiques sont réunies et séchées sur de sulfate de magnésium anhydre. L'huile obtenue après filtration et évaporation du solvant, est purifiée par chromatographie sur colonne de gel de silice, avec un mélange de dichlorométhane/méthanol (95:5) employé comme éluant, pour donner 0,31 g du produit désiré sous forme d'une huile.
Rdt: 34%
PF= huile

**6.2- N-[2-(3-Diéthylamino-propyle)-N-méthyl-1,2,3,4-tétrahydroisoquinoline-7-sulfonamide**

A une solution de 0,20 g (0,00046 mole) du N-[2-(3-Diéthylamino-propyle)-N-méthyl-2-(2,2,2-trifluoroacétyl)-1,2,3,4-tétrahydroisoquinoline-7-sulfonamide dissous dans 10 ml de méthanol saturé en chlorure d'hydrogène est chauffé durant douze heures à 60°C. La solution est concentrée à sec et le résidu est traité avec de l'eau. La phase aqueuse est extraite plusieurs fois avec de l'éther éthylique. Les phases organiques sont réunies, séchées sur sulfate de magnésium anhydre et la solution est concentrée à sec, pour donner 0,02 g du produit désiré sous forme d'une huile.
Rdt: 12%
PF= huile

**1H-RMN (DMSO)** $\delta$ **(ppm):** 9,67 (1 H, s, NH), 7,7 (1 H, s), 7,68 (1 H, d), 7,5 (1 H, d), 4,4 (2H, s), 3,5-2,9 (12H, m), 2,7 (3H, s), 1,9 (2H, m), 1,15 (6H, t)

***Exemple 7. Ethanodioate (1:1) de 2-cyclohéxyl-N-[3-(diéthylamino)propyl]-1,2,3,4-tétrahydroisoquinoline-7-sulfonamide***

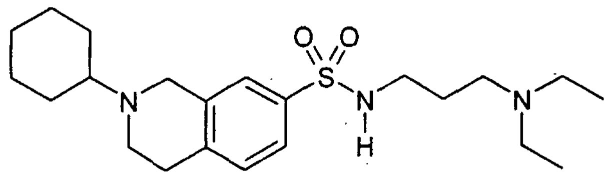

A une suspension de 1,70 g (0,0050 mole) de N-[3-(diéthylamino)propyl]-1,2,3,4-tétrahydroisoquinoline-7-sulfonamide (obtenue selon la méthode décrite à l'étape 3.2- de l'exemple 3) et 0,98 g (0,0014 mole) de cyclohexanone dans 50 ml de tétrahydrofurane, on ajoute 5 ml d'acide acétique. Le mélange est agité durant 3 heures à température ambiante et on ajoute 0,47 g (0.0075 mole) de cyanoborohydrure de sodium. On agite le mélange pendant la nuit. On concentre à sec. Le résidu est traité avec de l'eau et lavé avec de l'éther éthylique. La phase aqueuse est basifiée à pH= 10 et est extraite plusieurs fois avec de l'éther éthylique. Les phases organiques sont réunies et séchées sur sulfate de magnésium anhydre. L'huile obtenue est purifiée par chromatographie sur colonne de gel de silice, avec un mélange de dichlorométhane/méthanol (95:5) employé comme éluant. Le produit désiré (0,22 g; 11 %) est obtenu sous forme d'une huile.

L'huile précédente est dissoute dans 5 ml d'éthanol, puis une solution de 0,11 g (0,0012 mole) d'acide oxalique, dissous dans 5 ml d'éthanol, est ajoutée. Le précipité est filtré et lavé avec de l'éthanol froid. On obtient 0,32 g du produit désiré comme un solide blanc

Rdt: 97%
PF= 76-83°C.
**$^1$H-NMR (DMSO-$d_6$) δ(ppm):** 7,6(2H,m), 7,4 (1H,m), 4,2 (2H,s), 3,3 (H, m), 3,0 (H, m), 2,7 (H, m), 2,4 (H, m), 2,0 (H, m), 1,7 (H, m), 1,4 (H, m), 1,1 (H, m)

***Exemple 8- 1-Aminopropylpyrroline***

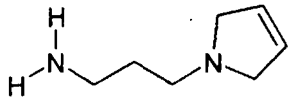

**8.1-1-(2-cyanoéthyl)pyrroline**

A une solution de 1,00g (0.0014 mole) de pyrroline dans méthanol sont ajoutés à 0°C, 0,95 ml (0.0014 moles) d'acrylonitrile. Le mélange est agité pendant la nuit à température ambiante et est concentré à sec pour donner 1,65 g du produit désiré sous forme d'huile.
Rdt: 93 %
PF= huile

**8.2-1-(3-aminopropyl)pyrroline**

A une solution de 1,00g (0,0089 mole) de 1-(2-cianoéthyl)pyrroline dans 20 ml de dichlorométhane, on ajoute 0,024 g d'hydrure de diisobutylalluminium 1 M dans le toluène. Le mélange est agité pendant la nuit à température ambiante. La solution est traitée avec du sulfate de sodium décahydrate. On agite pendant demi-heure. Les phases inorganiques sont filtrées et le filtrat est concentré à sec pour obtenir 1,00 g d'une huile incolore.
Rdt: 97%
PF= huile
**$^1$H-NMR ($CD_3Cl$) δ (ppm):** 5,75 (2H, s), 3,47 (4H, s), 2,75 (2H, t), 2,56 (2H, t), 1,69 (2H, q)
Le tableau ci-après illustre les structures chimiques et les propriétés physiques de quelques composés selon l'invention. Les micro-analyses élémentaires et les spectres RMN, IR ou de masse confirment les structures des composés obtenus.
Dans le tableau, pour les composés de formule I « P.F. » correspond au point de fusion.

**Tableau 1**

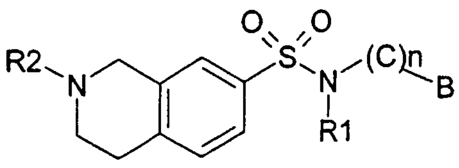

I

| N° | R2 | -(C)n- | R1 | B | P.F. (°C) | sel |
|---|---|---|---|---|---|---|
| 1. | H | $-(CH_2)_3-$ | H | $-N(C_2H_5)_2$ | 97-102 | Chlorhydrate |
| 2. | H | $-(CH_2)_2-$ | H | 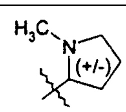 | 209-212 | Chlorhydrate |
| 3. | $CH_3$ | $-(CH_2)_3-$ | H | $-N(C_2H_5)_2$ | 58-62 | Oxalate |
| 4. | H | $-(CH_2)_3-$ | $CH_3$ | $-N(C_2H_5)_2$ | amorphe | Chlorhydrate |

(suite)

| N° | R2 | -(C)n- | R1 | B | P.F. (°C) | sel |
|---|---|---|---|---|---|---|
| 5. | 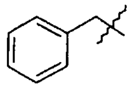 | $-(CH_2)_3-$ | H | $-N(C_2H_5)_2$ | 91-95 | Oxalate |
| 6. | 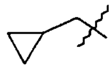 | $-(CH_2)_3-$ | H | $-N(C_2H_5)_2$ | 98-103 | Oxalate |
| 7. | 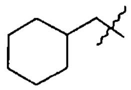 | $-(CH_2)_3-$ | H | $-N(C_2H_5)_2$ | 144-147 | Oxalate |
| 8. | H | $-(CH_2)_3-$ | H | 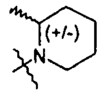 | 65-70 | Chlorhydrate |
| 9. | H | $-(CH_2)_3-$ | H | 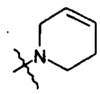 | >250 | Chlorhydrate |
| 10. | 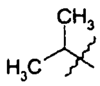 | $-(CH_2)_3-$ | H | $-N(C_2H_5)_2$ | 54-59 | Oxalate |
| 11. | 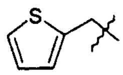 | $-(CH_2)_3-$ | H | $-N(C_2H_5)_2$ | 109-115 | Oxalate |
| 12. | 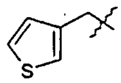 | $-(CH_2)_3-$ | H | $-N(C_2H_5)_2$ | 89-95 | Oxalate |
| 13. | H | $-(CH_2)_3-$ | H | 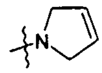 | 228-231 | Chlorhydrate |
| 14. | 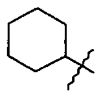 $-(CH_2)_3-$ | $-(CH_2)_3-$ | H | $-N(C_2H_5)_2$ | 55-60 | Oxalate |
| 15. | 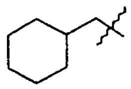 | $-(CH_2)_2-$ | H | 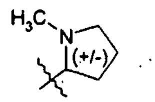 | 142-148 | Oxalate |
| 16. | $-CH_3$ | $-(CH_2)_3-$ | H | 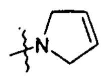 | 99-104 | Oxalate |
| 17. | H | $-(CH_2)_3-$ | H | 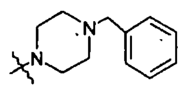 | 244-249 | Chlorhydrate |
| 18. | H | $-(CH_2)_3-$ | H | 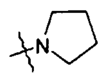 | 237-241 | Chlorhydrate |
| 19. | H | $-(CH_2)_3-$ | H | 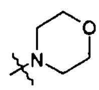 | 238-245 | Chlorhydrate |
| 20. | H | $-(CH_2)_3-$ | H | $-N(CH_3)_2$ | 230-234 | Chlorhydrate |
| 21. | 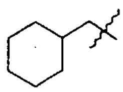 | $-(CH_2)_3-$ | H | 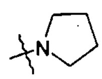 | 130-141 | Oxalate |
| 22. | 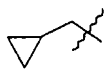 | $-(CH_2)_2-$ | H | 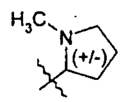 | 50-60 | Oxalate |

(suite)

| N° | R2 | -(C)n- | R1 | B | P.F. (°C) | sel |
|---|---|---|---|---|---|---|
| 23. | 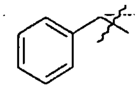 | $-(CH_2)_2-$ | H | 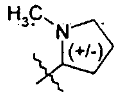 | 77-82 | Oxalate |
| 24. | 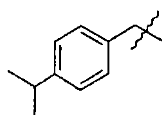 | $-(CH_2)_2-$ | H | 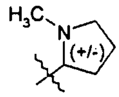 | 103-110 | Oxalate |
| 25. | 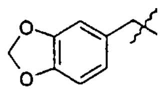 | $-(CH_2)_2-$ | H | 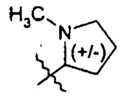 | 106-109 | Oxalate |
| 26. | H | $-(CH_2)_2-$ | H | 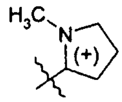 | 114-117 | Chlorhydrate |
| 27. | H | $-(CH_2)_2-$ | H | 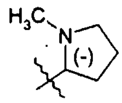 | 115-117 | Chlorhydrate |
| 28. | 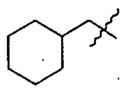 | $-(CH_2)_2-$ | H | 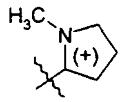 | 134-140 | Oxalate |
| 29. | 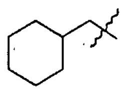 | $-(CH_2)_2-$ | H | 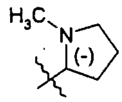 | 133-138 | Oxalate |
| 30. | 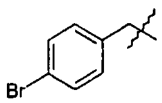 | $-(CH_2)_2-$ | H | 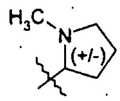 | 86-110 | Oxalate |
| 31. | 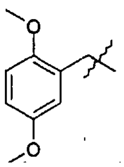 | $-(CH_2)_2-$ | H | 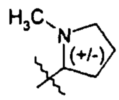 | 87-90 | Oxalate |
| 32. | 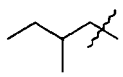 | $-(CH_2)_2-$ | H | 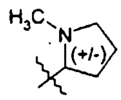 | 66-77 | Oxalate |
| 33. | 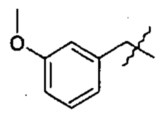 | $-(CH_2)_2-$ | H | 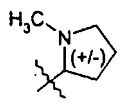 | 80-107 | Oxalate |
| 34. | 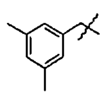 | $-(CH_2)_2-$ | H | 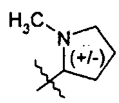 | 121-125 | Oxalate |

Les composés de l'invention de formule I ont fait l'objet d'essais pharmacologiques qui ont montré leur intérêt comme substances actives en thérapeutique.

Plus particulièrement, les composés de l'invention sont des antagonistes du récepteur de l'histamine du type $H_3$. Les récepteurs du type $H_3$ sont connus de l'homme du métier et leur intérêt en thérapeutique a été décrit dans la littérature («Histamine H3 receptor antagonists» Exp. Opinion Ther. Patents (2000) 10 (7) :1045-1055).
Ainsi, les composés de l'invention de formule I ont été soumis à un test d'affinité *in vitro* sur le récepteur natif de l'histamine du type $H_3$ dans une préparation membranaire de cerveau de rat adulte par la liaison spécifique de $[^3H]$-N-$\alpha$-méthylhis-

tamine à ce récepteur, selon les méthodes décrites par Korte, A. et al. dans Biochem. Biophys. Res. Commun. 168, 979-986(1990) et par West, R.E. Jr. et al. dans Mol. Pharmacol. 38, 610-613(1990).

Les $K_i$ des composés de l'invention vis-à-vis des récepteurs $H_3$ se situent entre 0,1 nM et 5,0 μMet plus particulièrement le (+/-)-2-(cyclohéxylmethyl)-N-[2-(1-méthylpyrrolidin-2-yl)éthyl]-1,2,3,4-tétrahydroisoquinoline-7-sulfonamide (composé 15; Table 1) présente une Ki de 0,3 nM.

Les composés de l'invention de formule I ont été soumis aussi à un test de formation de cAMP, sur le récepteur humain de l'histamine du type $H_3$ transfecté dans cellules CHO, par la inhibition du agonisme provoqué par la liaison spécifique de R-α-méthylhistamine à ce récepteur, selon les méthodes décrites par Lovenberg, T.W. et al. dans J. Pharmacol. Exp. Ther. 293, 771-778(2000).

Les $CI_{50}$ des composés de l'invention vis-à-vis des récepteurs $H_3$ se situent entre 0,1 nM et 5,0 μM.

A titre d'exemple, le composé 15, inclus dans le tableau 1, présente une $CI_{50}$<10 nM, faisant usage d'un kit EIA (Amersham) pour mesurer la formation de cAMP, sur le récepteur humain de l'histamine du type $H_3$ transfecté dans cellules CHO, par la inhibition du agonisme provoqué par la liaison spécifique de R-α-méthylhistamine à ce récepteur.

Les composés selon l'invention ont une activité sélective du récepteur de l'histamine du type $H_3$. Effectivement, les composés présentent un Ki supérieur à 7,0 μM dans le test d'affinité *in vitro* sur le récepteur natif de l'histamine du type $H_1$ dans une préparation membranaire de cerveau de rat adulte par la liaison spécifique de [$^3$H]-pirilamine à ce récepteur, selon la méthode décrite par Liu Y.Q. et al. dans, J.Pharmacol. Exp. Ther. 268, 959 (1994).

D'autre part, les composés de l'invention de formule I ont fait l'objet de tests *in vivo* montrant leur aptitude à réduire la prise de nourriture chez le rat à jeun 24h.

Les expériences ont été réalisées sur des rats Wistar. Les rats ont été placés individuellement dans des cages en plastique transparentes 48x26, 5x21,5 cm. Ces cages ont été placées dans une pièce isolée de tout bruit, à une température de 20 à 22°C, avec un cycle de lumière allant de 7h du matin à 7h du soir, les rats ayant libre accès à l'eau et à la nourriture.

Avant de réaliser l'expérience, les rats ont été mis à jeun durant 24h avec toutefois accès à l'eau *ad libitum.* Le jour de l'expérience, le véhicule ou le composé selon la présente invention est administré, par voie i.p. ou p.o., 15 ou 30 minutes avant la mise à disposition d'une quantité connue de nourriture (30g).
Chaque heure, durant 6 heures, la quantité de nourriture ingérée par le rat est mesurée.

Il a été montré que les $DA_{50}$ (mg/kg i.p. ou p.o.) des composés de l'invention vis-à-vis de la prise de nourriture peuvent être inférieurs à 10. Par exemple, le (+/-)-2-(4-isopropylbenzyl)-N-[2-(1-méthylpyrrolidin-2-yl)éthyl]-1,2,3,4-tétrahydroîsoquinoline-7-sulfonamide (composé 24; Table 1) diminue de 54% la prise de nourriture pendant la première heure après l'administration de 10 mg/kg par voie i.p. du produit.

Les résultats des tests montrent que les composés de l'invention permettent de réduire la prise de nourriture chez l'animal. Ainsi, ils permettent de contrôler la prise de poids, de traiter l'obésité ou d'aider à la perte de poids, chez l'animal, mais également chez l'homme.

Ainsi, selon un autre de ses aspects, l'invention a pour objet des médicaments qui comprennent un composé de formule I ou un sel d'addition de ce dernier à un acide pharmaceutiquement acceptable ou encore un hydrate ou un solvat du composé de formule I.

Ces médicaments trouvent leur emploi en thérapeutique, notamment dans le traitement des pathologies dans lesquelles un antagoniste du récepteur de l'histamine du type $H_3$ apporte un bénéfice thérapeutique. Notamment de telles pathologies sont l'obésité et le diabète. Egalement, ces composés peuvent être employés dans le traitement des maladies du système nerveux central telles que troubles de la vigilance et du sommeil, la narcolepsie, la maladie d'Alzheimer et autres démences, la maladie de Parkinson, les troubles de l'attention chez l'enfant hyperkinétique, les troubles de la mémoire et de l'apprentissage, l'épilepsie, la schizophrénie, les troubles cognitifs modérés, la dépression et l'anxiété. Les états de dépression et d'anxiété comprennent, par exemple, les anxiétés de type anticipatoire (avant intervention chirurgicale, avant traitement dentaire, etc), l'anxiété causée par la dépendance ou le sevrage d'alcool, de drogue, la manie, les désordres affectifs saisonniers, les migraines et les nausées. Ils peuvent être aussi utilisés dans le traitement des dysfonctionnements sexuels, des vertiges et du mal des voyages. L'utilisation des composés selon l'invention pour la préparation d'un médicament destiné à traiter les pathologies ci-dessus mentionnées fait partie intégrante de l'invention.
Selon un autre de ses aspects, la présente invention concerne des compositions pharmaceutiques comprenant, en tant que principe actif, au moins un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un ou plusieurs excipients pharmaceutiquement acceptables. Les dits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule I ci-dessus, ou son sel, solvat ou hydrate éventuel, peut être administré sous forme unitaire

d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Afin d'obtenir l'effet prophylactique ou thérapeutique désiré, la dose de principe actif peut varier entre 0,1 μg et 50 mg par kg de poids du corps et par jour. Chaque dose unitaire peut contenir de 0,1 à 1000 mg, de préférence de 1 à 500 mg, de principe actif en combinaison avec un excipient pharmaceutique. Cette dose unitaire peut être administrée 1 à 5 fois par jour de façon à administrer un dosage journalier de 0,5 à 5000 mg, de préférence de 1 à 2500mg

Il peut y avoir des cas particuliers où des dosages plus élevés ou plus faibles sont appropriés. De tels dosages appartiennent également à l'invention. Selon la pratique habituelle, le dosage approprié à chaque patient est déterminé par le médecin selon le mode d'administration, le poids et la réponse dudit patient.

A titre d'exemple, une forme unitaire d'administration d'un composé selon l'invention :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention selon un autre de ses aspects, divulgue également une méthode de traitement des pathologies ci-dessus indiquées qui comprend l'administration, à un patient, d'une dose efficace d'un composé selon l'invention ou un des ses sels pharmaceutiquement acceptables ou hydrates ou solvats.

## Revendications

1. Composé de formule I :

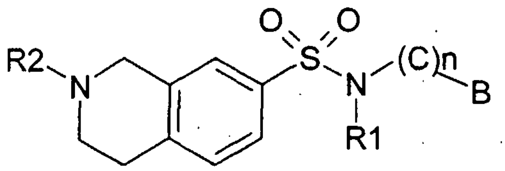

I

dans laquelle:

**n** peut prendre une valeur de 1 à 6 ;
**-(C)n-** représente un groupe $C_{1-6}$ alkylidène; éventuellement substitué par 1 à 4 substituants choisis parmi un atome d'halogène, un groupe hydroxy, nitro, cyano, amino, $C_{1-3}$ monoalkylamino, $C_{2-6}$ dialkylamino ou $C_{1-3}$ alcoxy ;
**R1** représente

- Un atome d'hydrogène
- Un groupe $C_{1-6}$ alkyle

**R2** représente

- Un atome d'hydrogène,
- Un groupe $C_{1-6}$ alkyle ou $C_{3-6}$ cycloalkyle éventuellement substitués par 1 à 4 substituants choisis parmi un atome d'halogène, un groupe hydroxy, nitro, cyano, amino, $C_{1-3}$ monoalkylamino, $C_{2-6}$ dialkylamino,

$C_{1-2}$ perhalogénoalkyle, $C_{1-3}$ halogénoalkyle, $C_{1-3}$ alcoxy, $C_{3-6}$ cycloalkyle, un hétéroaryle monocyclique tel qu'un thiényle, furyle ou pyrrolyle ou un aryle, tel qu'un phényle ou un naphtyle; l'aryle étant éventuellement substitué par 1 à 4 substituants choisis parmi un atome d'halogène, un groupe hydroxy, nitro, cyano, amino, $C_{1-3}$ monoalkylamino, $C_{2-6}$ dialkylamino, $C_{1-3}$ alkyle, $C_{1-2}$ perhalogénoalkyle, $C_{1-3}$ halogénoalkyle, $C_{1-3}$ alcoxy ou un groupe $C_{1-3}$ alkylidènedioxy ;

**B** représente

• NR3R4,

- R3 et R4 représentant, indépendamment l'un de l'autre, un groupe $C_{1-6}$ alkyle, un atome d'hydrogène; ou
- R3 et R4 représentent ensemble un groupe $C_{1-6}$ alkylidène, un groupe $C_{2-8}$ alkenylidène, un groupe $C_{1-3}$ alkylidène-O-$C_{1-3}$alkylidène, ou un groupe $C_{1-3}$ alkylidène-N(R5)-$C_{1-3}$ alkylidène où R5 représente un atome d'hydrogène, un groupe $C_{1-3}$ alkyle ou $C_{1-6}$ alkylcarbonyle, ces groupes $C_{1-3}$alkyle et $C_{1-6}$ alkylcarbonyle pouvant être substitués par un atome d'halogène, un groupe hydroxy, $C_{1-3}$ alcoxy, nitro, cyano ou amino; ou

• un aminocycle, lié par un carbone au groupe -NR1-(C)n-, choisi parmi aziridine, azétidine, pyrrolidine, pipéridine et morpholine ;

les groupes R3, R4, ainsi que l'aminocycle étant éventuellement substitués par 1 à 4 substituants choisis parmi un phényle, un benzyle, un atome d'halogène, un groupe hydroxy, nitro, cyano, amino, $C_{1-3}$ monoalkylamino, $C_{2-6}$ dialkylamino, $C_{1-3}$ alkyle, ou $C_{1-3}$ alcoxy ; et l'atome d'azote éventuellement substitué par un $C_{1-3}$ alkyle, à l'état de base ou de sel d'addition à un acide, ainsi qu'à l'état d'hydrate ou de solvat,
à l'exclusion du composé dans lequel $R_1$ et $R_2$ représentent des atomes d'hydrogène, B représente un groupe diméthylamino et -(C)n- représente un groupe éthylidène.

**2.** Composé selon la revendication 1 **caractérisé en ce que** :

- **n** est égal à 2,3 ou 4; et
- **R1** représente un atome d'hydrogène ou un groupe $C_{1-2}$ alkyle; et
- **R2** représente un atome d'hydrogène, un groupe $C_{1-4}$ alkyle ou $C_{5-6}$ cycloalkyle éventuellement substitués par 1 à 4 substituants choisis parmi un phényle, un groupe $C_{3-6}$cycloalkyle, $C_{1-2}$ perhalogénoalkyle, $C_{1-3}$ halogénoalkyle ou $C_{1-3}$ alcoxy ; le phényle étant éventuellement substitués par 1 à 4 substituants choisis parmi un atome d'halogène, un groupe hydroxy, nitro, cyano, amino, $C_{1-3}$ monoalkylamino, $C_{2-6}$ dialkylamino, $C_{1-3}$ alkyle, $C_{1-2}$ perhalogénoalkyle, $C_{1-3}$ halogénoalkyle, $C_{1-3}$ alcoxy ou un groupe $C_{1-3}$ alkylidènedioxy ; et
- **B** représente

• NR3R4,

- R3 et R4 représentant, indépendamment l'un de l'autre, un groupe $C_{1-4}$ alkyle; ou
- lorsque R3 et R4 représentent ensemble un groupe $C_{1-6}$ alkylidène, un groupe $C_{2-8}$ alkenylidène, $C_{1-3}$ alkylidène-O-$C_{1-3}$ alkylidène, ou $C_{1-3}$ alkylidène-N(R5)-$C_{1-3}$ alkylidène , B représente un groupe :

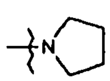 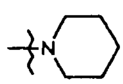 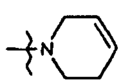 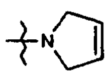 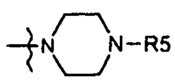 . 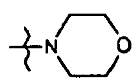 ;

• ou un aminocycle lié par un carbone au groupe -NR1-(C)n-, choisi parmi aziridine, azetidine, pyrrolidine, pipéridine et morpholine;les groupes R3, R4 et R5 ainsi que l'aminocycle étant éventuellement substitués ; à l'état de base ou de sel d'addition à un acide, ainsi qu'à l'état d'hydrate ou de solvat.

**3.** Composé selon la revendication 2 **caractérisé en ce que** lorsque B représente NR3R4 et R3 et R4 forment ensemble un groupe $C_{1-6}$ alkylidène, un groupe $C_{2-8}$ alkenylidène, $C_{1-3}$ alkylidène-O-$C_{1-3}$ alkylidène ou $C_{1-3}$ alkylidène-N (R5)-$C_{1-3}$ alkylidène ou lorsque **B** représente un aminocycle alors, B est choisi parmi les groupes suivants:

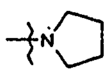 ou 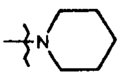 ou 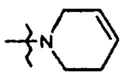 ou 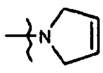 ou 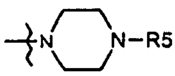 ou 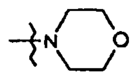

ou

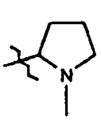 ou 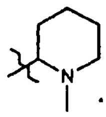 .

**4.** Composé selon la revendication 1, 2 ou 3 **caractérisé en ce qu'**il consiste en le :

- N-[3-(diéthylamino)propyl]-1,2,3,4-tétrahydroisoquinoline-7-sulfonamide;
- (+/-)-N-[2-(1-méthylpyrrolidin-2-yl)éthyl]-1,2,3,4-tétrahydroisoquinoline-7-sulfonamide;
- N-[3-(diéthylamino)propyl]-2-méthyl-1,2,3,4-tétrahydroisoquinoline-7-sulfonamide;
- N-[3-(diéthylamino)propyl]-N-méthyl-1,2,3,4-tétrahydroisoquinoline-7-sulfonamide;
- 2-benzyl-N-[3-(diéthylamino)propyl]-1,2,3,4-tétrahydroisoquinoline-7-sulfonamide;
- 2-(cyclopropylméthyl)-N-[3-(diéthylamino)propyl]-1,2,3,4-tétrahydroisoquinoline-7-sulfonamide;
- 2-(cyclohéxylmethyl)-N-[3-(diéthylamino)propyl]-1,2,3,4-tétrahydroisoquinoline-7-sulfonamide;
- (+/-)-N-[3-(2-méthylpiperidin-1-yl)propyl]-1,2,3,4-tétrahydroisoquinoline-7-sulfonamide;
- N-[3-(3,6-dihydropyridin-1(2*H*)-yl)propyl]-1,2,3,4-tétrahydroisoquinoline-7-sulfonamide;
- N-[3-(diéthylamino)propyl]-2-isopropyl-1,2,3,4-tétrahydroisoquinoline-7-sulfonamide;
- N-[3-(diéthylamino)propyl]-2-(2-thiénylméthyl)-1,2,3,4-tetrahydroisoquinoline-7-sulfonamide;
- N-[3-(diéthylamino)propyl]-2-(3-thiénylméthyl)-1,2,3,4-tetrahydroisoquinoline-7-sulfonamide;
- N-[3-(2,5-dihydro-1*H*-pyrrol-1-yl)propyl]-1,2,3,4-tétrahydroisoquinoline-7-sulfonamide;
- 2-cyclohéxyl-N-[3-(diéthylamino)propyl]-1,2,3,4-tétrahydroisoquinoline-7-sulfonamide;
- (+/-)-2-(cyclohéxylmethyl)-N-[2-(1-méthylpyrrolidin-2-yl)éthyl]-1,2,3,4-tétrahydroisoquinoline-7-sulfonamide;
- N-[3-(2,5-dihydro-1*H*-pyrrol-1-yl)propyl]-2-méthyl-1,2,3,4-tétrahydroisoquinoline-7-sulfonamide;
- N-[3-(4-benzylpiperazin-1-yl)propyl]-1,2,3,4-tétrahydroisoquinoline-7-sulfonamide;
- N-(3-pyrrolidin-1 -ylpropyl)- 1,2,3,4-tétrahyd roisoquinoline-7-sulfonam ide;
- N-(3-morpholin-4-ylpropyl)-1,2,3,4-tétrahydroisoquinoline-7-sulfonamide;
- N-[3-(diméthylamino)propyl]-1,2,3,4-tétrahydroisoquinoline-7-sulfonamide;
- 2-(cyclohéxylmethyl)-N-(3-pyrrolidin-1-ylpropyl)-1,2,3,4-tétrahydroisoquinoline-7-sulfonamide;
- (+/-)-2-(cyclopropylméthyl)-N-[2-(1-méthylpyrrolidin-2-yl)éthyl]-1,2,3,4-tétrahydroisoquinoline-7-sulfonamide;
- (+/-)-2-benzyl-N-[2-(1-méthylpyrrolidin-2-yl)éthyl]-1,2,3,4-tétrahydroisoquinoline-7-sulfonamide;
- (+/-)-2-(4-isopropylbenzyl)-N-[2-(1-méthylpyrrolidin-2-yl)éthyl]-1,2,3,4-tétrahydroisoquinoline-7-sulfonamide;
- (+/-)-2-(1,3-benzodioxol-5-ylméthyl)-N-[2-(1-méthylpyrrolidin-2-yl)éthyl]-1,2,3,4-tétrahydroisoquinoline-7-sulfonamide;
- (+)-N-[2-(1-méthylpyrrolidin-2-yl)éthyl]-1,2,3,4-tétrahydroisoquinoline-7-sulfonamide;
- (-)-N-[2-(1-méthylpyrrolidin-2-yl)éthyl]-1,2,3,4-tétrahydroisoquinoline-7-sulfonamide;
- (+)-2-(cyclohéxylmethyl)-N-[2-(1-méthylpyrrolidin-2-yl)éthyl]-1,2,3,4-tétrahydroisoquinoline-7-sulfonamide; et
- (-)-2-(cyclohéxylmethyl)-N-[2-(1-méthylpyrrolidin-2-yl)éthyl]-1,2,3,4-tétrahydroisoquinoline-7-sulfonamide à l'état de base ou de sel d'addition à un acide, ainsi qu'à l'état d'hydrate ou de solvat.
- (+/-)-2-(4-bromo-benzyl)-)-*N*-[2-(1-méthylpyrrolidin-2-yl)éthyl]-1,2,3,4-tétrahydroisoquinoline-7-sulfonamide
- (+/-)-2-(2,5-dimétoxy-benzyl)-)-*N*-[2-(1-méthylpyrrolidin-2-yl)éthyl]-1,2,3,4-tétrahydroisoquinoline-7-sulfonamide
- (+/-)-2-(2-méthyl-butyl)-)-*N*-[2-(1-méthylpyrrolidin-2-yl)éthyl]-1,2,3,4-tétrahydroisoquinoline-7-sulfonamide
- (+/-)-2-(3-méthoxy-benzyl)-)-*N*-[2-(1-méthylpyrrolidin-2-yl)éthyl]-1,2,3,4-tétrahydroisoquinoline-7-sulfonamide
- (+/-)-2-(3,5-diméthylbenzyl)-)-*N*-[2-(1-méthylpyrrolidin-2-yl)éthyl]-1,2,3,4-tétrahydroisoquinoline-7-sulfonamide

**5.** Composé de formule II:

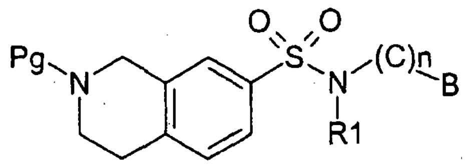

II

dans laquelle Pg représente un atome d'hydrogène ou un groupe protecteur et -(C)n - et R1 sont tels que définis dans la revendication 1, et B représente :

- NR3R4, où R3 et R4 sont tels que définis à la revendication 1
- ou un aminocycle lié par un carbone au groupe -NR1-(C)n-, choisi parmi aziridine, azetidine, pyrrolidine, piperidine et morpholine ;

les groupes R3, R4, ainsi que l'aminocycle étant éventuellement substitués tel que défini à la revendication 1, à l'exclusion des composés dans lesquels R1 représente un atome d'hydrogène, B représente un groupe diméthylamino et $-(C)_n-$ représente un groupe éthylidène.

**6.** Composition pharmaceutique contenant un composé de formule I, selon l'une quelconque des revendications 1 à 4, ou son sel, solvat ou hydrate, et au moins un excipient pharmaceutique.

**7.** Utilisation d'un composé de formule I selon l'une quelconque des revendications 1 à 4, ou son sel, solvat ou hydrate, pour la préparation d'un médicament destiné à traiter l'obésité et le diabète.

**8.** Utilisation d'un composé de formule I selon l'une quelconque des revendications 1 à 4, ou son sel, solvat ou hydrate, pour la préparation d'un médicament destiné au traitement des maladies du système nerveux central telles que troubles de la vigilance et du sommeil, la narcolepsie, la maladie d'Alzheimer et autres démences, la maladie de Parkinson, les troubles de l'attention chez l'enfant hyperkinétique, les troubles de la mémoire et de l'apprentissage, l'épilepsie, la schizophrénie, les troubles cognitifs modérés, la dépression, l'anxiété, les dysfonctionnements sexuels, les vertiges et le mal des voyages.

## Claims

**1.** Compound of formula I:

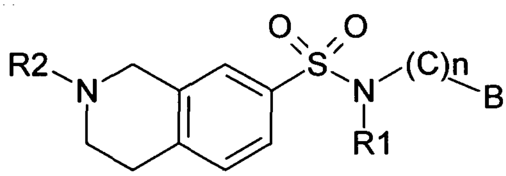

I

in which:

**n** can represent a value between 1 and 6;
**-(C)n-** represents a $C_{1-6}$ alkylidene group optionally substituted with 1 to 4 substituents chosen from a halogen atom, and a hydroxyl, nitro, cyano, amino, $C_{1-3}$ monoalkylamino, $C_{2-6}$ dialkylamino or $C_{1-3}$ alkoxy group;
**R1** represents

- a hydrogen atom,
- a $C_{1-6}$ alkyl group;

**R2** represents

- a hydrogen atom,
- a $C_{1-6}$ alkyl or $C_{3-6}$ cycloalkyl group optionally substituted with 1 to 4 substituents chosen from a halogen atom, a hydroxyl, nitro, cyano, amino, $C_{1-3}$ monoalkylamino, $C_{2-6}$ dialkylamino, $C_{1-2}$ perhaloalkyl, $C_{1-3}$ haloalkyl, $C_{1-3}$ alkoxy or $C_{3-6}$ cycloalkyl group, a monocyclic heteroaryl such as a thienyl, furyl or pyrrolyl, or an aryl, such as a phenyl or a naphthyl; the aryl being optionally substituted with 1 to 4 substituents chosen from a halogen atom, a hydroxyl, nitro, cyano, amino, $C_{1-3}$ monoalkylamino, $C_{2-6}$ dialkylamino, $C_{1-3}$ alkyl, $C_{1-2}$ perhaloalkyl, $C_{1-3}$ haloalkyl or $C_{1-3}$ alkoxy group or a $C_{1-3}$ alkylidenedioxy group;

**B** represents

- NR3R4,

  - R3 and R4 represent, independently of one another, a $C_{1-6}$ alkyl group, or a hydrogen atom; or
  - R3 and R4 together represent a $C_{1-6}$ alkylidene group, a $C_{2-8}$ alkenylidene group, a $C_{1-3}$ alkylidene-O-$C_{1-3}$ alkylidene group, or a $C_{1-3}$ alkylidene-N(R5)-$C_{1-3}$ alkylidene group where R5 represents a hydrogen atom, or a $C_{1-3}$ alkyl or $C_{1-6}$ alkylcarbonyl group, it being possible for these $C_{1-3}$ alkyl and $C_{1-6}$ alkylcarbonyl groups to be substituted with a halogen atom, or a hydroxyl, $C_{1-3}$ alkoxy, nitro, cyano or amino group; or

- an aminocycle, linked via a carbon to the group -NR1-(C)n-, chosen from aziridine, azetidine, pyrrolidine, piperidine and morpholine;

the groups R3 and R4 and also the aminocycle being optionally substituted with 1 to 4 substituents chosen from a phenyl, a benzyl, a halogen atom, and a hydroxyl, nitro, cyano, amino, $C_{1-3}$ monoalkylamino, $C_{2-6}$ dialkylamino, $C_{1-3}$ alkyl or $C_{1-3}$ alkoxy group; and the nitrogen atom optionally substituted with a $C_{1-3}$ alkyl, in the form of a base or of an addition salt with an acid, and also in the form of a hydrate or of a solvate,
with the exclusion of the compound in which $R_1$ and $R_2$ represent hydrogen atoms, B represents a dimethylamino group and -(C)n- represents an ethylidene group.

**2.** Compound according to Claim 1 **characterized in that**:

- **n** is equal to 2, 3 or 4; and
- **R1** represents a hydrogen atom or a $C_{1-2}$ alkyl group; and
- **R2** represents a hydrogen atom, or a $C_{1-4}$ alkyl or $C_{5-6}$ cycloalkyl group optionally substituted with 1 to 4 substituents chosen from a phenyl, and a $C_{3-6}$ cycloalkyl, $C_{1-2}$ perhaloalkyl, $C_{1-3}$ haloalkyl or $C_{1-3}$ alkoxy group; the phenyl being optionally substituted with 1 to 4 substituents chosen from a halogen atom, a hydroxyl, nitro, cyano, amino, $C_{1-3}$ monoalkylamino, $C_{2-6}$ dialkylamino, $C_{1-3}$ alkyl, $C_{1-2}$ perhaloalkyl, $C_{1-3}$ haloalkyl or $C_{1-3}$ alkoxy group or a $C_{1-3}$ alkylidenedioxy group; and
- **B** represents

  - NR3R4,

    - R3 and R4 represent, independently of one another, a $C_{1-4}$ alkyl group; or
    - when R3 and R4 together represent a $C_{1-6}$ alkylidene group, a $C_{2-8}$ alkenylidene group, a $C_{1-3}$ alkylidene-O-$C_{1-3}$ alkylidene group or a $C_{1-3}$ alkylidene-N(R5)-$C_{1-3}$ alkylidene group , B represents a group:

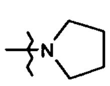 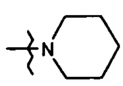 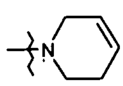 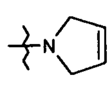 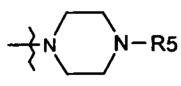 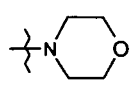 ;

  - or an aminocycle linked via a carbon to the group -NR1-(C)n, chosen from aziridine, azetidine, pyrrolidine, piperidine and morpholine;

the groups R3, R4 and R5 and also the aminocycle being optionally substituted;

in the form of a base or of an addition salt with an acid, and also in the form of a hydrate or of a solvate.

3. Compound according to Claim 2, **characterized in that** when B represents NR3R4 and R3 and R4 together form a $C_{1-6}$ alkylidene group, a $C_{2-8}$ alkenylidene group, a $C_{1-3}$ alkylidene-O-$C_{1-3}$ alkylidene group or a $C_{1-3}$ alkylidene-N(R5)-$C_{1-3}$ alkylidene group, or when **B** represents an aminocycle, then B is chosen from the following groups:

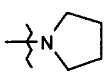 or 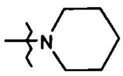 or 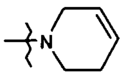 or 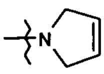 or 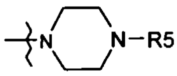 or 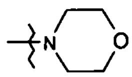

or

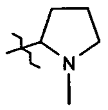 or 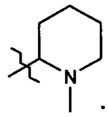.

4. Compound according to Claim 1, 2 or 3, **characterized in that** it consists of:

- N-[3-(diethylamino)propyl]-1,2,3,4-tetrahydroisoquinoline-7-sulfonamide;
- (+/-)-N-[2-(1-methylpyrrolidin-2-yl)ethyl]-1,2,3,4-tetrahydroisoquinoline-7-sulfonamide;
- N-[3-(diethylamino)propyl]-2-methyl-1,2,3,4-tetrahydroisoquinoline-7-sulfonamide;
- N-[3-(diethylamino)propyl]-N-methyl-1,2,3,4-tetrahydroisoquinoline-7-sulfonamide;
- 2-benzyl-N-[3-(diethylamino)propyl]-1,2,3,4-tetrahydroisoquinoline-7-sulfonamide;
- 2-(cyclopropylmethyl)-N-[3-(diethylamino)propyl]-1,2,3,4-tetrahydroisoquinoline-7-sulfonamide;
- 2-(cyclohexylmethyl)-N-[3-(diethylamino)propyl]-1,2,3,4-tetrahydroisoquinoline-7-sulfonamide;
- (+/-)-N-[3-(2-methylpiperidin-1 -yl)propyl]-1,2,3,4-tetrahydroisoquinoline-7-sulfonamide;
- N-[3-(3,6-dihydropyridin-1 (2*H*)-yl)propyl]-1,2,3,4-tetrahydroisoquinoline-7-sulfonamide;
- N-(3-(diethylamino)propyl]-2-isopropyl-1,2,3,4-tetrahydroisoquinoline-7-sulfonamide;
- N-[3-(diethylamino)propyl]-2-(2-thienylmethyl)-1,2,3,4-tetrahydroisoquinoline-7-sulfonamide;
- N-[3-(diethylamino)propyl]-2-(3-thienylmethyl)-1,2,3,4-tetrahydroisoquinoline-7-sulfonamide;
- N-[3-(2,5-dihydro-1*H*-pyrrol-1-yl)propyl]-1,2,3,4-tetrahydroisoquinoline-7-sulfonamide;
- 2-cyclohexyl-N-[3-(diethylamino)propyl]-1,2,3,4-tetrahydroisoquinoline-7-sulfonamide;
- (+/-)-2-(cyclohexylmethyl)-N-[2-(1-methylpyrrolidin-2-yl)ethyl]-1,2,3,4-tetrahydroisoquinoline-7-sulfonamide;
- N-[3-(2,5-dihydro-1*H*-pyrrol-1-yl)propyl]-2-methyl-1,2,3,4-tetrahydroisoquinoline-7-sulfonamide;
- N-[3-(4-benzylpiperazin-1-yl)propyl]-1,2,3,4-tetrahydroisoquinoline-7-sulfonamide;
- N-(3-pyrrolidin-1-ylpropyl)-1,2,3,4-tetrahydroisoquinoline-7-sulfonamide;
- N-(3-morpholin-4-ylpropyl)-1,2,3,4-tetrahydroisoquinoline-7-sulfonamide;
- N-[3-(dimethylamino)propyl]-1,2,3,4-tetrahydroisoquinoline-7-sulfonamide;
- 2-(cyclohexylmethyl)-N-(3-pyrrolidin-1-ylpropyl)-1,2,3,4-tetrahydroisoquinoline-7-sulfonamide;
- (+/-)-2-(cyclopropylmethyl)-N-[2-(1-methylpyrrolidin-2-yl)ethyl]-1,2,3,4-tetrahydroisoquinoline-7-sulfonamide;
- (+/-)-2-benzyl-N-[2-(1-methylpyrrolidin-2-yl)ethyl]-1,2,3,4-tetrahydroisoquinoline-7-sulfonamide;
- (+/-)-2-(4-isopropylbenzyl)-N-(2-(1-methylpyrrolidin-2-yl)ethyl]-1,2,3,4-tetrahydroisoquinoline-7-sulfonamide;
- (+/-)-2-(1,3-benzodioxol-5-ylmethyl)-N-[2-(1-methylpyrrolidin-2-yl)ethyl]-1,2,3,4-tetrahydroisoquinoline-7-sulfonamide;
- (+)-N-[2-(1-methylpyrrolidin-2-yl)ethyl]-1,2,3,4-tetrahydroisoquinoline-7-sulfonamide;
- (-)-N-[2-(1-methylpyrrolidin-2-yl)ethyl]-1,2,3,4-tetrahydroisoquinoline-7-sulfonamide;
- (+)-2-(cyclohexylmethyl)-N-[2-(1-methylpyrrolidin-2-yl)ethyl]-1,2,3,4-tetrahydroisoquinoline-7-sulfonamide;
- (-)-2-(cyclohexylmethyl)-N-[2-(1-methylpyrrolidin-2-yl)ethyl]-1,2,3,4-tetrahydroisoquinoline-7-sulfonamide, in the form of a base or of an addition salt with an acid, and also in the form of a hydrate or a solvate
- (+/-)-2-(4-bromobenzyl)-*N*-[2-(1-methylpyrrolidin-2-yl)ethyl]-1,2,3,4-tetrahydroisoquinoline-7-sulfonamide
- (+/-)-2-(2,5-dimethoxybenzyl)-*N*-[2-(1-methylpyrrolidin-2-yl)ethyl]-1,2,3,4-tetrahydroisoquinoline-7-sulfonamide
- (+/-)-2-(2-methylbutyl)-*N*-[2-(1-methylpyrrolidin-2-yl)ethyl]-1,2,3,4-tetrahydroisoquinoline-7-sulfonamide
- (+/-)-2-(3-methoxybenzyl)-)-*N*-[2-(1-methylpyrrolidin-2-yl)ethyl]-1,2,3,4-tetrahydroisoquinoline-7-sulfonamide
- (+/-)-2-(3,5-dimethylbenzyl)-*N*-[2-(1-methylpyrrolidin-2-yl)ethyl]-1,2,3,4-tetrahydroisoquinoline-7-sulfonamide.

5. Compound of formula II:

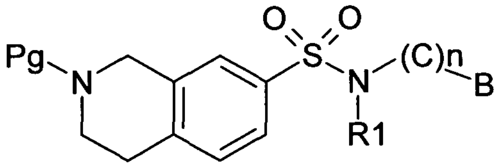

II

in which Pg represents a hydrogen atom or a protective group, and -(C)n- and R1 are as defined in claim 1, and B represents:

- NR3R4, where R3 and R4 are as defined in claim 1;
- or an aminocycle linked via a carbon to the group -NR1-(C)n-, chosen from aziridine, azetidine, pyrrolidine, piperidine and morpholine;

the groups R3 and R4 and also the aminocycle being optionally substituted as defined in claim 1, with the exclusion of the compounds in which R1 represents a hydrogen atom, B represents a dimethylamino group and -(C)n- represents an ethylidene group.

**6.** Pharmaceutical composition containing a compound of formula I, according to any one of Claims 1 to 4, or the salt, solvate or hydrate thereof, and at least one pharmaceutical excipient.

**7.** Use of a compound of formula I according to any one of Claims 1 to 4, or the salt, solvate or hydrate thereof, for the preparation of a medicament for use in treating obesity and diabetes.

**8.** Use of a compound of formula I according to any one of Claims 1 to 4, or the salt, solvate or hydrate thereof, for the preparation of a medicament for use in the treatment of central nervous system diseases such as vigilance and sleep disorders, narcolepsy, Alzheimer's disease and other dementias, Parkinson's disease, attention disorders in hyperkinetic children, memory and learning disorders, epilepsy, schizophrenia, moderate cognitive disorders, depression, anxiety, sexual dysfunction, dizziness and travel sickness.

**Patentansprüche**

**1.** Verbindung der Formel I:

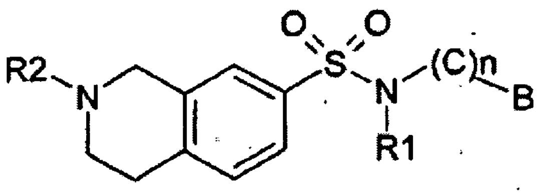

I

worin:

**n** einen Wert von 1 bis 6 annehmen kann;
**-(C)n-** für eine $C_{1-6}$-Alkylidengruppe steht, die gegebenenfalls durch 1 bis 4 Substituenten, die aus einem Halogenatom und einer Hydroxy-, Nitro-, Cyano-, Amino-, $C_{1-3}$-Monoalkylamino-, $C_{2-6}$-Dialkylamino- oder $C_{1-3}$-Alkoxygruppe ausgewählt sind, substituiert ist;
**R1** für

- ein Wasserstoffatom,
- eine $C_{1-6}$-Alkylgruppe

steht;
**R2** für

- ein Wasserstoffatom,
- eine $C_{1-6}$-Alkyl- oder $C_{3-6}$-Cycloalkylgruppe, die gegebenenfalls durch 1 bis 4 Substituenten, die aus einem Halogenatom, einer Hydroxy-, Nitro-, Cyano-, Amino-, $C_{1-3}$-Monoalkylamino-, $C_{2-6}$-Dialkylamino-, $C_{1-2}$-Perhalogenalkyl-, $C_{1-3}$-Halogenalkyl-, $C_{1-3}$-Alkoxy- oder $C_{3-6}$-Cycloalkylgruppe, einem monocyclischen Heteroaryl, wie einem Thienyl, Furyl oder Pyrrolyl, oder einem Aryl, wie einem Phenyl oder einem Naphthyl, ausgewählt sind, substituiert ist; wobei das Aryl gegebenenfalls durch 1 bis 4 Substituenten, die aus einem Halogenatom, einer Hydroxy-, Nitro-, Cyano-, Amino-, $C_{1-3}$-Monoalkylamino-, $C_{2-6}$-Dialkylamino-, $C_{1-3}$-Alkyl-, $C_{1-2}$-Perhalogenalkyl-, $C_{1-3}$-Halogenalkyl- oder $C_{1-3}$-Alkoxygruppe oder einer $C_{1-3}$-Alkylidendioxygruppe ausgewählt sind, substituiert ist;

steht;
**B** für

- NR3R4,

  - wobei R3 und R4 unabhängig voneinander für eine $C_{1-6}$-Alkylgruppe oder ein Wasserstoffatom stehen; oder
  - R3 und R4 zusammen für eine $C_{1-6}$-Alkyliden-gruppe, eine $C_{2-8}$-Alkenylidengruppe, eine $C_{1-3}$-Alkyliden-O-$C_{1-3}$-alkylidengruppe oder eine $C_{1-3}$-Alkyliden-N(R5)-$C_{1-3}$-alkylidengruppe stehen, wobei R5 für ein Wasserstoffatom oder eine $C_{1-3}$-Alkyl- oder $C_{1-6}$-Alkylcarbonylgruppe steht, wobei diese $C_{1-3}$-Alkyl- und $C_{1-6}$-Alkyl-carbonylgruppen durch ein Halogenatom oder eine Hydroxy-, $C_{1-3}$-Alkoxy-, Nitro-, Cyano- oder Aminogruppe substituiert sein können; oder

- einen Aminocyclus, der über ein Kohlenstoffatom an die Gruppe -NR1-(C)n- gebunden ist und aus Aziridin, Azetidin, Pyrrolidin, Piperidin und Morpholin ausgewählt ist;

steht; wobei die Gruppen R3 und R4 sowie der Aminocyclus gegebenenfalls durch 1 bis 4 Substituenten, die aus einem Phenyl, einem Benzyl, einem Halogenatom und einer Hydroxy-, Nitro-, Cyano-, Amino-, $C_{1-3}$-Monoalkylamino-, $C_{2-6}$-Dialkylamino-, $C_{1-3}$-Alkyl- oder $C_{1-3}$-Alkoxygruppe ausgewählt sind, substituiert sind und das Stickstoffatom gegebenenfalls durch ein $C_{1-3}$-Alkyl substituiert ist, in Basen- oder Säureadditionssalzform sowie in Hydrat- oder Solvatform,
unter Ausschluss der Verbindung, in der $R_1$ und $R_2$ für Wasserstoffatome stehen, B für eine Dimethylaminogruppe steht und -(C)n- für eine Ethylidengruppe steht.

**2.** Verbindung nach Anspruch 1, **dadurch gekennzeichnet, dass**:

- **n** gleich 2, 3 oder 4 ist; und
- **R1** für ein Wasserstoffatom oder eine $C_{1-2}$-Alkylgruppe steht; und
- **R2** für ein Wasserstoffatom oder eine $C_{1-4}$-Alkyl- oder $C_{5-6}$-Cycloalkylgruppe, die gegebenenfalls durch 1 bis 4 Substituenten, die aus einem Phenyl und einer $C_{3-6}$-Cycloalkyl-, $C_{1-2}$-Perhalogenalkyl-, $C_{1-3}$-Halogenalkyl- oder $C_{1-3}$-Alkoxygruppe ausgewählt sind, substituiert ist; wobei das Phenyl gegebenenfalls durch 1 bis 4 Substituenten, die aus einem Halogenatom, einer Hydroxy-, Nitro-, Cyano-, Amino-, $C_{1-3}$-Monoalkylamino-, $C_{2-6}$-Dialkylamino-, $C_{1-3}$-Alkyl-, $C_{1-2}$-Perhalogenalkyl-, $C_{1-3}$-Halogenalkyl- oder $C_{1-3}$-Alkoxygruppe oder einer $C_{1-3}$-Alkylidendioxygruppe ausgewählt sind, substituiert ist; steht; und
- **B** für

  - NR3R4,

    - wobei R3 und R4 unabhängig voneinander für eine $C_{1-4}$-Alkylgruppe stehen; oder
    - dann, wenn R3 und R4 zusammen für eine $C_{1-6}$-Alkylidengruppe, eine $C_{2-8}$-Alkenylidengruppe, eine $C_{1-3}$-Alkyliden-O-$C_{1-3}$-alkylidengruppe oder eine $C_{1-3}$-Alkyliden-N(R5)-$C_{1-3}$-alkylidengruppe stehen, B für eine Gruppe:

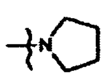 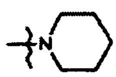 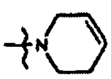 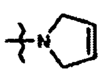 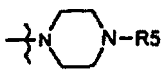 . 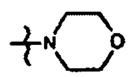 ;

steht;

• oder einen Aminocyclus, der über ein Kohlenstoffatom an die Gruppe -NR1-(C)n- gebunden ist und aus Aziridin, Azetidin, Pyrrolidin, Piperidin und Morpholin ausgewählt ist; wobei die Gruppen R3, R4 und R5 sowie der Aminocyclus gegebenenfalls substituiert sind;
steht;

in Basen- oder Säureadditionssalzform sowie in Hydrat- oder Solvatform.

**3.** Verbindung nach Anspruch 2, **dadurch gekennzeichnet, dass** B für NR3R4 steht und R3 und R4 zusammen eine $C_{1-6}$-Alkylidengruppe, eine $C_{2-8}$-Alkenyliden-gruppe, eine $C_{1-3}$-Alkyliden-O-$C_{1-3}$-alkylidengruppe oder eine $C_{1-3}$-Alkyliden-N(R5)-$C_{1-3}$-alkylidengruppe bilden oder dann, wenn **B** für einen Aminocyclus steht, B aus den folgenden Gruppen ausgewählt ist:

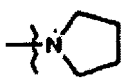 oder 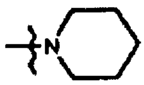 oder 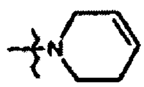 oder 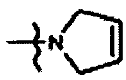

oder

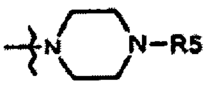 oder 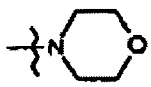 oder 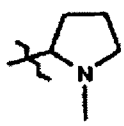 oder 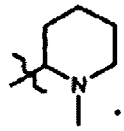.

**4.** Verbindung nach Anspruch 1, 2 oder 3, **dadurch gekennzeichnet, dass** sie besteht aus:

• N-[3-(Diethylamino)propyl]-1,2,3,4-tetrahydroisochinolin-7-sulfonamid;
• (+/-)-N-[2-(1-Methylpyrrolidin-2-yl)ethyl]-1,2,3,4-tetrahydroisochinolin-7-sulfonamid;
• N-[3-(Diethylamino)propyl]-2-methyl-1,2,3,4-tetrahydroisochinolin-7-sulfonamid;
• N-[3-(Diethylamino)propyl]-N-methyl-1,2,3,4-tetrahydroisochinolin-7-sulfonamid;
• 2-Benzyl-N-[3-(diethylamino)propyl]-1,2,3,4-tetrahydroisochinolin-7-sulfonamid;
• 2-(Cyclopropylmethyl)-N-[3-(diethylamino)propyl]-1,2,3,4-tetrahydroisochinolin-7-sulfonamid;
• 2-(Cyclohexylmethyl)-N-[3-(diethylamino)propyl]-1,2,3,4-tetrahydroisochinolin-7-sulfonamid;
• (+/-)-N-[3-(2-Methylpiperidin-1-yl)propyl]-1,2,3,4-tetrahydroisochinolin-7-sulfonamid;
• N-[3-(3,6-Dihydropyridin-1(2*H*)-yl)propyl]-1,2,3,4-tetrahydroisochinolin-7-sulfonamid:
• N-[3-(Diethylamino)propyl]-2-isopropyl-1,2,3,4-tetrahydroisochinolin-7-sulfonamid;
• N-[3-(Diethylamino)propyl]-2-(2-thienylmethyl)-1,2,3,4-tetrahydroisochinolin-7-sulfonamid;
• N-[3-(Diethylamino)propyl]-2-(3-thienylmethyl)-1,2,3,4-tetrahydroisochinolin-7-sulfonamid;
• N-[3-(2,5-Dihydro-1H-pyrrol-1-yl)propyl]-1,2,3,4-tetrahydroisochinolin-7-sulfonamid;
• 2-Cyclohexyl-N-[3-(diethylamino)propyl]-1,2,3,4-tetrahydroisochinolin-7-sulfonamid;
• (+/-)-2-(Cyclohexylmethyl)-N-[2-(1-methylpyrrolidin-2-yl)ethyl]-1,2,3,4-tetrahydroisochinolin-7-sulfonamid;
• N-[3-(2,5-Dihydro-1H-pyrrol-1-yl)propyl]-2-methyl-1,2,3,4-tetrahydroisochinolin-7-sulfonamid;
• N-[3-(4-Benzylpiperazin-1-yl)propyl]-1,2,3,4-tetrahydroisochinolin-7-sulfonamid;
• N-(3-Pyrrolidin-1-ylpropyl)-1,2,3,4-tetrahydroisochinolin-7-sulfonamid;
• N-(3-Morpholin-4-ylpropyl)-1,2,3,4-tetrahydroisochinolin-7-sulfonamid;
• N-[3-(Dimethylamino)propyl]-1,2,3,4-tetrahydroisochinolin-7-sulfonamid;
• 2-(Cyclohexylmethyl)-N-(3-pyrrolidin-1-ylpropyl)-1,2,3,4-tetrahydroisochinolin-7-sulfonamid;
• (+/-)-2-(Cyclopropylmethyl)-N-[2-(1-methylpyrrolidin-2-yl)ethyl]-1,2,3,4-tetrahydroisochinolin-7-sulfonamid;
• (+/-)-2-Benzyl-N-[2-(1-methylpyrrolidin-2-yl)-ethyl]-1,2,3,4-tetrahydroisochinolin-7-sulfonamid;
• (+/-)-2-(4-Isopropylbenzyl)-N-[2-(1-methylpyrrolidin-2-yl)ethyl]-1,2,3,4-tetrahydroisochinolin-7-sulfonamid;
• (+/-)-2-(1,3-Benzodioxol-5-ylmethyl)-N-[2-(1-methylpyrrolidin-2-yl)ethyl]-1,2,3,4-tetrahydroisochinolin-7-sulfonamid;
• (+)-N-[2-(1-Methylpyrrolidin-2-yl)ethyl]-1,2,3,4-tetrahydroisochinolin-7-sulfonamid;
• (-)-N-[2-(1-Methylpyrrolidin-2-yl)ethyl]-1,2,3,4-tetrahydroisochinolin-7-sulfonamid;
• (+)-2-(Cyclohexylmethyl)-N-[2-(1-methylpyrrolidin-2-yl)ethyl]-1,2,3,4-tetrahydroisochinolin-7-sulfonamid;
• (-)-2-(Cyclohexylmethyl)-N-[2-(1-methylpyrrolidin-2-yl)ethyl]-1,2,3,4-tetrahydroisochinolin-7-sulfonamid in Basen- oder Säureadditionssalzform sowie in Hydrat- oder Solvatform;
• (+/-)-2-(4-Brombenzyl)-N-[2-(1-methylpyrrolidin-2-yl)ethyl]-1,2,3,4-tetrahydroisochinolin-7-sulfonamid;

• (+/-)-2-(2,5-Dimethoxybenzyl)-N-[2-(1-methylpyrrolidin-2-yl)ethyl]-1,2,3,4-tetrahydroisochinolin-7-sulfonamid;
• (+/-)-2-(2-Methylbutyl)-N-[2-(1-methylpyrrolidin-2-yl)ethyl]-1,2,3,4-tetrahydroisochinolin-7-sulfonamid;
• (+/-)-2-(3-Methoxybenzyl)-N-[2-(1-methylpyrrolidin-2-yl)ethyl]-1,2,3,4-tetrahydroisochinolin-7-sulfonamid
• (+/-)-2-(3,5-Dimethylbenzyl)-N-[2-(1-methylpyrrolidin-2-yl)ethyl]-1,2,3,4-tetrahydroisochinolin-7-sulfonamid.

**5.** Verbindung der Formel II:

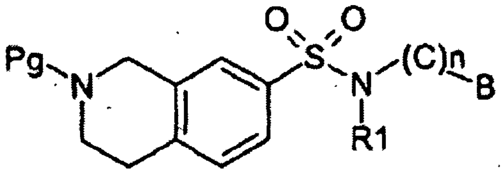

II

worin Pg für ein Wasserstoffatom oder eine Schutzgruppe steht und -(C)n- und R1 die in Anspruch 1 angegebene Bedeutung besitzen und B für:

• NR3R4, wobei R3 und R4 die in Anspruch 1 angegebene Bedeutung besitzen;
• oder einen Aminocyclus, der über ein Kohlenstoffatom an die Gruppe -NR1-(C)n- gebunden ist und aus Aziridin, Azetidin, Pyrrolidin, Piperidin und Morpholin ausgewählt ist;

steht; wobei die Gruppen R3 und R4 sowie der Aminocyclus gegebenenfalls wie in Anspruch 1 definiert substituiert sind, unter Ausschluss der Verbindungen, in denen R1 für ein Wasserstoffatom steht, B für eine Dimethylaminogruppe steht und -(C)n- für eine Ethylidengruppe steht.

**6.** Pharmazeutische Zusammensetzung, enthaltend eine Verbindung der Formel I nach einem der Ansprüche 1 bis 4 oder ein Salz, Solvat oder Hydrat davon und mindestens einen pharmazeutischen Hilfsstoff.

**7.** Verwendung einer Verbindung der Formel I nach einem der Ansprüche 1 bis 4 oder eines Salzes, Solvats oder Hydrats davon zur Herstellung eines Arzneimittels zur Behandlung von Obesitas und Diabetes.

**8.** Verwendung einer Verbindung der Formel I nach einem der Ansprüche 1 bis 4 oder eines Salzes, Solvats oder Hydrats davon zur Herstellung eines Arzneimittels zur Behandlung von Erkrankungen des Zentralnervensystems wie Aufmerksamkeits- und Schlafstörungen, Narkolepsie, Alzheimer-Krankheit und anderen Demenzen, Parkinson-Krankheit, hyperkinetischem Syndrom des Kindesalters, Gedächtnis- und Lernstörungen, Epilepsie, Schizophrenie, moderaten kognitiven Störungen, Angst, sexuellen Dysfunktionen, Schwindel und Reisekrankheit.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03055848 A
- WO 2004019935 A
- WO 02076925 A


### Littérature non-brevet citée dans la description


- Protective groups in Organic Synthesis. John Wiley & Sons, Inc, 1999
- Histamine H3 receptor antagonists. *Exp. Opinion Ther. Patents,* 2000, vol. 10 (7), 1045-1055
- **KORTE, A. et al.** *Biochem. Biophys. Res. Commun.,* 1990, vol. 168, 979-986
- **WEST, R.E. JR. et al.** *Mol. Pharmacol.,* 1990, vol. 38, 610-613
- **LOVENBERG, T.W. et al.** *J. Pharmacol. Exp. Ther.,* 2000, vol. 293, 771-778
- **LIU Y.Q. et al.** *J.Pharmacol. Exp. Ther.,* 1994, vol. 268, 959